# EUROPEAN PATENT APPLICATION

(11) **EP 3 677 213 A1**
(43) Date of publication of application: **08.07.2020**
(21) Application number: 18885537.3
(22) Date of filing: 10.09.2018
(51) Int. Cl.: A61B 90/20, A61B 34/00, G02B 21/00

(54) **MEDICAL CONTROL DEVICE AND MEDICAL OBSERVATION SYSTEM**

(30) Priority: 06.12.2017 JP 2017234283
(71) Applicant: Sony Olympus Medical Solutions Inc., Hachioji-shi, Tokyo 192-0904 (JP)
(72) Inventor: KATSUKI, Shinji, Hachioji-shi Tokyo 192-0904 (JP); KOBAYASHI, Motoaki, Hachioji-shi Tokyo 192-0904 (JP); ISHIKAWA, Tomonori, Hachioji-shi Tokyo 192-0904 (JP); SEGAWA, Kazunori, Hachioji-shi Tokyo 192-0904 (JP)
(74) Representative: D Young & Co LLP
(86) International application number: PCT/JP2018/033440
(87) International publication number: WO 2019/111473

(57) **Abstract**

Provided is a medical control apparatus including an imaging control unit that adjusts an inward angle which is an angle formed by an imaging direction in a first imaging device that captures a medical image for the right eye and an imaging direction in a second imaging device that captures a medical image for the left eye to the inward angle corresponding to a display device that displays the medical image for the right eye and the medical image for the left eye.

## Description

### Techinical Field

The present disclosure relates to a medical control apparatus and a medical observation system.

### Background Art

In recent years, there is a case, in medical fields, of using a medical observation apparatus capable of enlarged observation of an observation target such as an affected site in order to support microsurgery such as neurosurgery. Examples of the medical observation apparatus include a medical observation apparatus equipped with an optical microscope and a medical observation apparatus equipped with an imaging device that functions as an electronic imaging microscope. Hereinafter, the medical observation apparatus equipped with the above-described optical microscope will be referred to as an "optical medical observation apparatus". In addition, hereinafter, a medical observation apparatus equipped with the above-described imaging device will be referred to as an "electronic imaging medical observation apparatus" or simply as a "medical observation apparatus".

With increased image quality in imaging devices and display devices that display captured images, an electronic imaging medical observation apparatus can obtain image quality equal to or higher than that of an optical medical observation apparatus. In addition, a user of an electronic imaging medical observation apparatus (for example, a medical worker such as a practitioner or an assistant of the practitioner) need not observe through an eyepiece of an optical microscope as in the case of using an optical medical observation apparatus, and thus it is possible to more freely move the position of the imaging device. Therefore, the use of the electronic imaging medical observation apparatus has an advantage that surgical operations can be supported more flexibly by moving the position of the imaging device, promoting the use of the electronic imaging medical observation apparatus in medical fields.

Under such circumstances, technologies concerning an electronic imaging medical observation apparatus have been developed. Examples of the above technologies include the technology described in Patent Literature 1 below.

### Citation List

### Patent Literature

Patent Literature 1: JP 2017-158970 A

### Disclosure of Invention

### Technical Problem

For example, there are cases, in a surgical operation field, where a plurality of display devices to display an image of a medical practice target patient captured by a medical observation apparatus is arranged at positions where each of a practitioner, an assistant, and a nurse can obtain a good view". Hereinafter, an image captured by a medical observation apparatus is referred to as a "medical image".

Some medical observation apparatuses include a plurality of imaging devices and have a function of capturing a medical image for the right eye and a medical image for the left eye. In addition, some display devices have a configuration corresponding to any types of display capable of 3D display, such as an eyeglass type that enables 3D display in cooperation with an eyeglass-type device, or a naked eye type that enables 3D display without cooperation with the eyeglass-type device. With the medical image for the right eye and the medical image for the left eye displayed on individual display screens of display devices, a viewer of the display screen can obtain a three-dimensional effect on the displayed medical images.

Here, in order to reproduce a natural three-dimensional effect with less fatigue, it is desirable to achieve a match between an inward angle (described below) in two imaging devices, that is, the device for capturing a medical image for the right eye and the device for capturing a medical image for the left eye and a convergence angle (described below) formed by the right eye and the left eye of the viewer of the display screen. In the present specification, achieving a match between the inward angle (described below) and the convergence angle (described below) has two meanings, that is, achieving a complete match between the inward angle (described below) and the convergence angle (described below), and achieving a smaller difference between the inward angle (described below) and the convergence angle (described below).

In a case where a plurality of display devices is arranged in a surgical operation field, the convergence angle (described below) varies for each of display devices due to various factors such as the location of the display device and the size of the display screen. Furthermore, along with the convergence angle (described below) changing for each of display devices, the inward angle (described below) for reproducing a natural three-dimensional effect with less fatigue would also vary for each of display devices. The greater the difference between the inward angle (described below) and the convergence angle (described below), the more the viewer of the medical image displayed on the display screen feels that the image is a flat image with no stereoscopic effect, or that the image is a distorted image with an excessive three-dimensional effect.

The present disclosure proposes a novel and improved medical control apparatus and a medical observation system capable of obtaining a medical image with a more natural three-dimensional effect.

### Solution to Problem

According to the present disclosure, there is provided a medical control apparatus including an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye.

Moreover, according to the present disclosure, there is provided a medical observation system including: a medical control apparatus including an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye; and a display device that displays the medical image for the right eye and the medical image for the left eye on a display screen. Advantageous Effects of Invention

According to the present disclosure, it is possible to obtain a medical image with a more natural three-dimensional effect.

Note that the above-described effects are not necessarily limited, and any of the effects described in the present specification, or other effects that can be known from the present specification, together with the above effects or instead of the above effects, may be obtained.

### Brief Description of Drawings

FIG. 1 is a view illustrating an example of a configuration of a medical observation system according to the present embodiment.
FIG. 2 is a view illustrating an example of a configuration of a medical observation apparatus according to the present embodiment.
FIG. 3 is a view illustrating an example of a configuration of an imaging device unit included in the medical observation apparatus according to the present embodiment.
FIG. 4 is a functional block diagram illustrating an example of a configuration of a medical observation apparatus according to the present embodiment.
FIG. 5 is a view illustrating a control method according to the present embodiment.
FIG. 6 is a view illustrating an example of an adapter according to the present embodiment that can adjust an inward angle to a convergence angle corresponding to a display device.
FIG. 7 is a view illustrating an example of a configuration of an adjustment mechanism according to the present embodiment.
FIG. 8 is a view illustrating an example of an imaging control process according to a control method of the present embodiment.
FIG. 9 is a view illustrating an example of an imaging control process according to the control method of the present embodiment.
FIG. 10 is a view illustrating the significance of arranging a plurality of imaging devices on each of a first plane and a second plane according to the present embodiment.
FIG. 11 is a view illustrating the significance of arranging a plurality of imaging devices on each of a first plane and a second plane according to the present embodiment.
FIG. 12 is a view illustrating the significance of arranging a plurality of imaging devices on each of the first plane and the second plane according to the present embodiment.
FIG. 13 is a view illustrating an example of an imaging control process according to the control method of the present embodiment.
FIG. 14 is a view illustrating an example of a display control process according to the present embodiment.
FIG. 15 is a view illustrating an example of a surgical operation field where the medical observation system according to the present embodiment is used.
FIG. 16A is a view illustrating an example of a first imaging device and a second imaging device selected by the imaging control process according to the present embodiment, and a medical image displayed on a display device.
FIG. 16B is a view illustrating an example of a first imaging device and a second imaging device selected by the imaging control process according to the present embodiment, and a medical image displayed on a display device.
FIG. 16C is a view illustrating an example of a first imaging device and a second imaging device selected by the imaging control process according to the present embodiment, and a medical image displayed on a display device.
FIG. 17 is a view conceptually illustrating an example of arrangement of display devices in the medical observation system according to the present embodiment.
FIG. 18A is a flowchart illustrating an example of a process according to the control method of the present embodiment.
FIG. 18B is a flowchart illustrating an example of a process according to the control method of the present embodiment.

### Best Mode for Carrying Out the Invention

Hereinafter, preferred embodiments of the present disclosure will be described in detail with reference to the accompanying drawings. In this specification and the drawings, components having substantially the same functional configuration are denoted by the same reference numerals, and redundant description is omitted.

In the following, description will be given in the following order.
1. Medical observation system according to the present embodiment and control method according to the present embodiment
2. Program according to the present embodiment

(Medical observation system according to the present embodiment, and control method according to the present embodiment)

Hereinafter, a control method according to the present embodiment will be described together with an example of a medical observation system according to the present embodiment.

Hereinafter, description will be given focusing mainly on a case where the medical observation apparatus according to the present embodiment performs the process according to the control method of the present embodiment, that is, the case where the medical observation apparatus according to the present embodiment functions as a medical control apparatus. Note that the device that functions as the medical control apparatus in the medical observation system according to the present embodiment is not limited to the medical observation apparatus according to the present embodiment. For example, in the medical observation system according to the present embodiment, a display device according to the present embodiment may perform a process according to the control method of the present embodiment and may function as a medical control apparatus. In the medical observation system according to the present embodiment, any device, such as a medical controller, capable of performing the process according to the control method of the present embodiment can function as a medical control apparatus.

### [1] Configuration of medical observation system

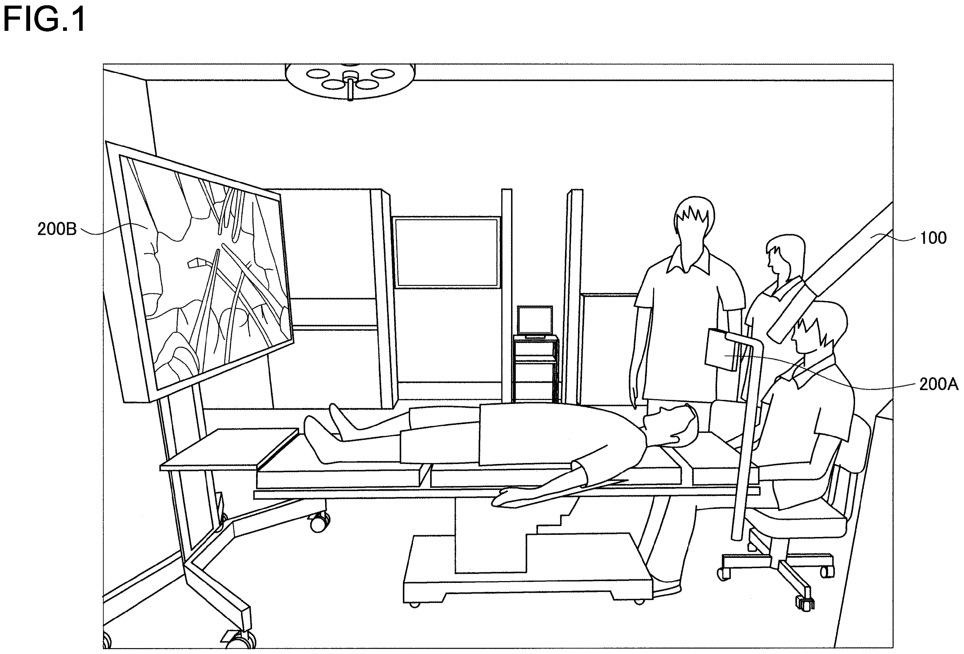

FIG. 1 is a view illustrating an example of a configuration of a medical observation system 1000 according to the present embodiment, illustrating an example of a surgical operation field in which the medical observation system 1000 is used. The medical observation system 1000 includes a medical observation apparatus 100 and display devices 200A and 200B, for example.

Note that the medical observation system according to the present embodiment is not limited to the example illustrated in FIG. 1.

For example, the medical observation system according to the present embodiment may further include a medical medical control apparatus (not illustrated) that performs a process according to the control method of the present embodiment and that controls various types of operation in the medical observation apparatus 100. As described below, the medical observation system 1000 illustrated in FIG. 1 is an example in which the medical observation apparatus 100 includes a control unit (described below) that performs a process according to the control method of the present embodiment, thereby enabling the medical observation apparatus 100 to have the function of the medical control apparatus (not illustrated).

Examples of the medical control apparatus (not illustrated) include any device that can perform a process according to the control method of the present embodiment, such as a "medical controller" or a "computer such as a server". Furthermore, the medical control apparatus (not illustrated) may be an integrated circuit (IC) that can be incorporated in the above device.

Furthermore, although FIG. 1 illustrates two display devices 200A and 200B, the number of display devices included in the medical observation system according to the present embodiment is not limited to two. For example, the medical observation system according to the present embodiment may include three or more display devices. Hereinafter, a plurality of display devices included in the medical observation system according to the present embodiment may be collectively or individually referred to as "display device 200".

Furthermore, the medical observation system according to the present embodiment may have a configuration including a plurality of the medical observation apparatuses 100. In a case where the plurality of medical observation apparatuses 100 is provided, a process according to the control method of the present embodiment is performed in each of the medical observation apparatuses 100. In a case where the medical observation system according to the present embodiment has a configuration including the plurality of medical observation apparatuses 100, the medical observation apparatus 100 and the display device 200 may be associated with each other one by one, or the plurality of medical observation apparatuses 100 may be associated with one display device 200. In a case where the plurality of medical observation apparatuses 100 is associated with one display device 200, the display device 200 performs switching operation or the like, for example, to select a medical image to be displayed on the display screen from the images captured by the plurality of medical observation apparatuses 100.

Hereinafter, each of devices included in the medical observation system 1000 will be described.

### [1-1] Display device 200

The display device 200 is a display unit in the medical observation system 1000 and corresponds to an external display device when viewed from the medical observation apparatus 100. The display device 200 displays various images such as a medical image (a moving image or a plurality of still images; the similar applies hereinafter) captured by the medical observation apparatus 100, an image related to a user interface (UI), or the like, on the display screen. Furthermore, the display device 200 has a configuration capable of performing 3D display by an arbitrary method. The display on the display device 200 is controlled by the medical observation apparatus 100 or a medical control apparatus (not illustrated), for example.

In the medical observation system 1000, the display device 200 is installed at any location visually recognizable by a person involved in the surgical operation including a practitioner in an operating room, such as a wall surface, a ceiling, or a floor surface of a surgical room, as illustrated in the display devices 200A and 200B in FIG. 1. Examples of the display device 200 include a liquid crystal display, an organic electro-luminescence (EL) display, a cathode ray tube (CRT) display, or the like.

Note that the display device 200 is not limited to the example described above.

For example, the display device 200 may be any wearable device used by a practitioner or the like worn on his body, such as a head-mounted display or an eyewear type device.

The display device 200 is driven by power supplied from an internal power supply such as a battery included in the display device 200 or power supplied from a connected external power supply.

### [1-2] Medical observation apparatus 100

The medical observation apparatus 100 is an electronic imaging medical observation apparatus. For example, in a case where the medical observation apparatus 100 is used at the time of surgery, the practitioner (an example of a user of the medical observation apparatus 100) observes a surgical site while viewing a medical image captured by the medical observation apparatus 100 and displayed on the display screen of the display device 200 and performs various treatments such as a procedure according to the surgical procedure, onto the surgical site.

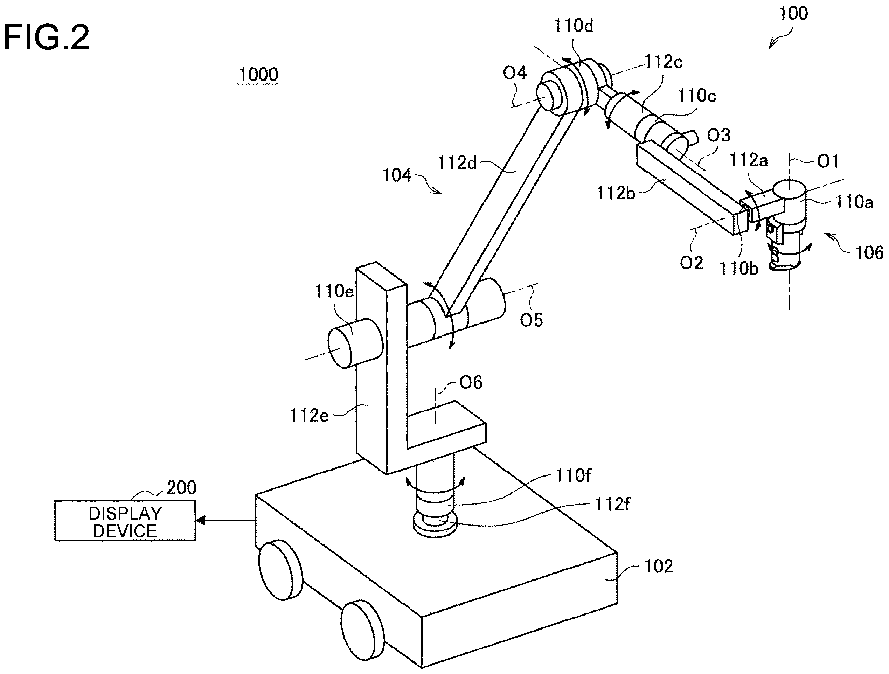

FIG. 2 is a view illustrating an example of a configuration of the medical observation apparatus 100 according to the present embodiment. FIG. 2 also illustrates the display device 200.

The medical observation apparatus 100 includes a base 102, an arm 104, and an imaging device unit 106, for example.

Although not illustrated in FIG. 1, the medical observation apparatus 100 may include, for example, one or two or more processors (not illustrated) including an arithmetic circuit such as a micro processing unit (MPU), and read only memory (ROM; not illustrated), random access memory (RAM; not illustrated), a recording medium (not illustrated), and a communication device (not illustrated). The medical observation apparatus 100 is driven by electric power supplied from an internal power supply such as a battery included in the medical observation apparatus 100 or by electric power supplied from a connected external power supply, for example.

The processor (not illustrated) functions as a control unit described below. The ROM (not illustrated) stores programs used by the processor (not illustrated) and control data such as calculation parameters. The RAM (not illustrated) temporarily stores a program executed by the processor (not illustrated), or the like.

The recording medium (not illustrated) functions as a storage unit (not illustrated) in the medical observation apparatus 100. The recording medium (not illustrated) stores various data such as data related to the control method according to the present embodiment and various applications. Here, examples of the recording medium (not illustrated) include a magnetic recording medium such as a hard disk, and nonvolatile memory such as flash memory. The recording medium (not illustrated) may be detachable from the medical observation apparatus 100.

The communication device (not illustrated) is a communication unit included in the medical observation apparatus 100 and has a role of performing wireless or wired communication with an external device such as the display device 200. Here, examples of the communication device (not illustrated) include an IEEE 802.15.1 port and a transmission/reception circuit (wireless communication), an IEEE 802.11 port and a transmission/reception circuit (wireless communication), a communication antenna and a radio frequency (RF) circuit (wireless communication), or a local area network (LAN) terminal and a transmission/reception circuit (wired communication).

### [1-2-1] Base 102

The base 102 provided as a base of the medical observation apparatus 100 is connected to one end of the arm 104 and supports the arm 104 and the imaging device unit 106.

The base 102 includes casters, for example, and the medical observation apparatus 100 is grounded to the floor via the casters. With the presence of casters, the medical observation apparatus 100 can easily move on the floor surface by the casters.

### [1-2-2] Arm 104

The arm 104 includes a plurality of links connected to each other by a joint.

The arm 104 supports the imaging device unit 106. The imaging device unit 106 supported by the arm 104 can move three-dimensionally, and the position and posture of the imaging device unit 106 after the move are held by the arm 104.

More specifically, the arm 104 includes, for example, a plurality of joints 110a, 110b, 110c, 110d, 110e, and 110f, and a plurality of links 112a, 112b, 112c, 112d, 112e, and 112f mutually pivotably joined to each other by the joints 110a, 110b, 110c, 110d, 110e, and 110f. The rotatable range of each of the joints 110a, 110b, 110c, 110d, 110e, and 110f is arbitrarily set in a design stage, a manufacturing stage, or the like so as to achieve a desired movement of the arm 104.

That is, the medical observation apparatus 100 illustrated in FIG. 1 achieves six degrees of freedom concerning the movement of the imaging device unit 106 by six rotation axes (a first axis O1, a second axis O2, a third axis O3, a fourth axis O4, a fifth arm O5, and a sixth arm O6) corresponding to the six joints 110a, 110b, 110c, 110d, 110e, and 110f, respectively forming the arm 104. More specifically, the medical observation apparatus 100 illustrated in FIG. 1 achieves a movement of six degrees of freedom, namely, three degrees of freedom of translation and three degrees of freedom of rotation.

Each of the joints 110a, 110b, 110c, 110d, 110e, and 110f includes an actuator (not illustrated), and each of the joints 110a, 110b, 110c, 110d, 110e, and 110f rotates around the corresponding rotation axis by driving of the actuator (not illustrated). The driving of the actuator (not illustrated) is controlled by a processor functioning as a control unit described below or by an external medical control apparatus (not illustrated), for example.

Rotation of each of the joints 110a, 110b, 110c, 110d, 110e, and 110f on the corresponding rotation axis by driving of the actuator (not illustrated) enables various types of operation of the arm 104, such as extension and contraction (folding) of the arm 104.

The joint 110a has a substantially columnar shape and supports the imaging device unit 106 (an upper end portion of the imaging device unit 106 in FIG. 1) at a distal end portion (lower end portion in FIG. 1) of the joint 110a so that the imaging device unit 106 is pivotable around a rotation axis (first axis O1) parallel to the central axis of the imaging device unit 106. Here, the medical observation apparatus 100 has a configuration in which the first axis O1 is aligned with the optical axis of the imaging device unit 106. That is, pivoting movement of the imaging device unit 106 about the first axis O1 illustrated in FIG. 1 allows a medical image captured by the imaging device unit 106 to be an image that is changed to rotate the visual field.

The link 112a is a substantially rod-like member, and fixedly supports the joint 110a. The link 112a extends in a direction orthogonal to the first axis O1, for example, and is connected to the joint 110b.

The joint 110b has a substantially columnar shape and supports the link 112a so as to be pivotable about a rotation axis (second axis O2) orthogonal to the first axis O1. Moreover, a link 112b is fixedly connected to the joint 110b.

The link 112b is a substantially rod-like member and extends in a direction orthogonal to the second axis O2. Moreover, each of the joints 110b and 110c is connected to the link 112b.

The joint 110c has a substantially columnar shape and supports the link 112b so as to be pivotable about a rotation axis (third axis O3) orthogonal to each of the first axis O1 and the second axis O2. Moreover, one end of the link 112c is fixedly connected to the joint 110c.

Here, pivoting of the distal end side of the arm 104 (the side on which the imaging device unit 106 is provided) about the second axis O2 and the third axis O3 enables the imaging device unit 106 to move so as to change the position of the imaging device unit 106 in the horizontal plane. That is, the medical observation apparatus 100 controls the rotation of the second axis O2 and the rotation about the third axis O3, making it possible to move the visual field of the medical image within a plane.

The link 112c is a member having a substantially columnar shape on one end and having a substantially rod-like shape on the other end. The joint 110c is fixedly connected to the one end of the link 112c such that the central axis of the joint 110c is aligned with the central axis of the substantially columnar shape. Furthermore, the joint 110d is connected to the other end of the link 112c.

The joint 110d has a substantially columnar shape and supports the link 112c so as to be pivotable about a rotation axis (a fourth axis O4) orthogonal to the third axis O3. The link 112d is fixedly connected to the joint 110d.

The link 112d is a substantially rod-like member and extends so as to be orthogonal to the fourth axis O4. One end of the link 112d is fixedly connected to the joint 110d so as to come in contact with the substantially columnar side surface of the joint 110d. Moreover, the joint 110e is connected to the other end of the link 112d (the end opposite to the side to which the joint 110d is connected).

The joint 110e has a substantially columnar shape and supports one end of the link 112d so as to be pivotable about a rotation axis (a fifth axis O5) parallel to the fourth axis O4. Moreover, the one end of a link 112e is fixedly connected to the joint 110e.

Here, the fourth axis O4 and the fifth axis O5 are rotation axes that can move the imaging device unit 106 in the perpendicular direction. Pivoting of the distal end side of the arm 104 (the side on which the imaging device unit 106 is provided) about the fourth axis O4 and the fifth axis O5 changes the position of the imaging device unit 106 in the perpendicular direction. Accordingly, pivoting of the distal end side of the arm 104 (the side on which the imaging device unit 106 is provided) about the fourth axis O4 and the fifth axis O5 makes it possible to change the distance between the imaging device unit 106 and an observation target such as a surgical site of a patient.

The link 112e is a member as a combination of a first member having a substantially L-shape in which one side extends in the vertical direction and the other side extends in the horizontal direction and a rod-like second member extending vertically downward from a site of the first member extending in the horizontal direction. The joint 110e is fixedly connected to a site of the first member of the link 112e extending in the vertical direction. Moreover, the joint 110f is connected to the second member of the link 112e.

The joint 110f has a substantially columnar shape and supports the link 112e so as to be pivotable about a rotation axis (sixth axis O6) parallel to the vertical direction. The link 112f is fixedly connected to the joint 110f.

The link 112f is a substantially rod-like member extending in the vertical direction. One end of the link 112f is connected to the joint 110f. The other end of the link 112f (the end opposite to the side to which the joint 110f is connected) is fixedly connected to the base 102.

With the arm 104 having the above-described configuration, the medical observation apparatus 100 achieves six degrees of freedom regarding the movement of the imaging device unit 106.

Note that the configuration of the arm 104 is not limited to the above example.

For example, each of the joints 110a, 110b, 110c, 110d, 110e, and 110f of the arm 104 may include a brake that regulates the rotation of each of the joints 110a, 110b, 110c, 110d, 110e, and 110f. Examples of the brake according to the present embodiment include any types of brakes, such as a mechanically driven brake and an electrically driven electromagnetic brake.

The driving of the brake is controlled by a processor functioning as a control unit described below, or an external medical control apparatus (not illustrated). The drive control of the brake allows the operation mode of the arm 104 to be set in the medical observation apparatus 100. The operation mode of the arm 104 includes, for example, a fixed mode and a free mode.

Here, the fixed mode according to the present embodiment is an operation mode in which the position and the posture of the imaging device unit 106 are fixed by regulation of the rotation of each of rotation axes provided on the arm 104, by the brake. When the arm 104 turns to the fixed mode, the operation state of the medical observation apparatus 100 turns to a fixed state in which the position and the posture of the imaging device unit 106 are fixed.

In addition, the free mode according to the present embodiment is an operation mode in which each of the rotation axes provided on the arm 104 turns into a free rotation state by releasing the brake. For example, the free mode enables adjustment of the position and posture of the imaging device unit 106 by direct operation of the practitioner. Here, the direct operation according to the present embodiment includes, for example, operation by a practitioner to hold the imaging device unit 106 by hand and to directly move the imaging device unit 106.

### [1-2-3] Imaging device unit 106

The imaging device unit 106 is supported by the arm 104, and captures an image of an observation target such as a surgical site of a patient. The imaging device unit 106 has a plurality of imaging devices and captures a medical image for the right eye and a medical image for the left eye. Imaging on the imaging device unit 106 is controlled by a processor functioning as a control unit described below, or an external medical control apparatus (not illustrated).

Each of the imaging devices constituting the imaging device unit 106 has a configuration corresponding to an electronic imaging microscope, for example.

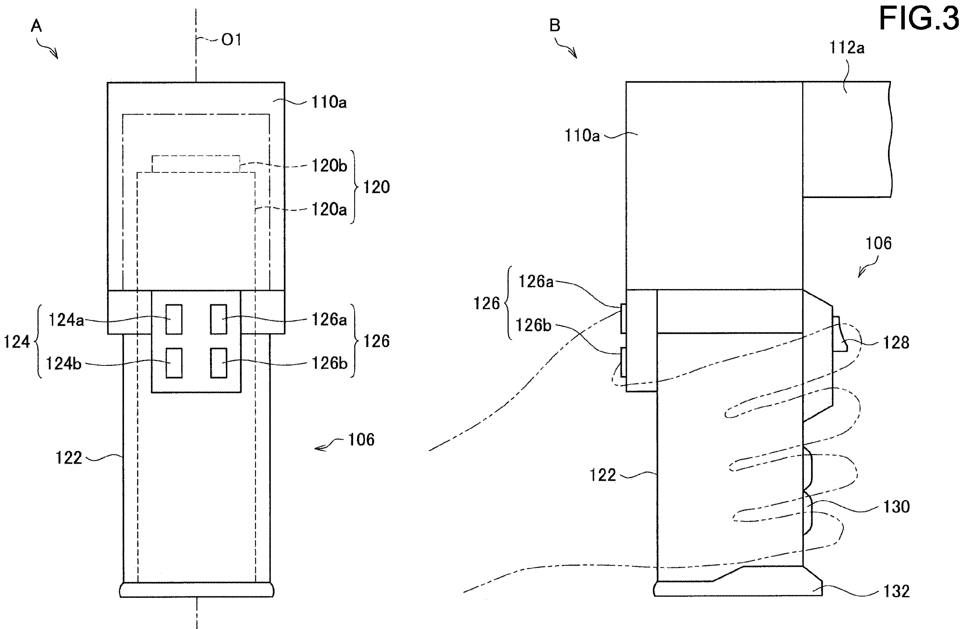

FIG. 3 is a view illustrating an example of a configuration of the imaging device unit 106 included in the medical observation apparatus 100 according to the present embodiment.

The imaging device unit 106 includes, for example, an imaging member 120 and a tubular member 122 having a substantially cylindrical shape. The imaging member 120 is provided inside the tubular member 122.

For example, a cover glass slip (not illustrated) for protecting the imaging member 120 is provided on an aperture surface of a lower end (a lower end in FIG. 3) of the tubular member 122.

Furthermore, for example, a light source (not illustrated) is provided inside the tubular member 122, and illumination light is emitted from the light source to a subject through a cover glass slip at the time of imaging. Reflected light (observation light) from the subject on which the illumination light is applied is incident on the imaging member 120 via the cover glass slip (not illustrated), whereby an image signal indicating the subject (image signal indicating the medial image) is obtained by the imaging member 120.

The imaging member 120 functions as a plurality of imaging devices. The number of imaging devices included in the imaging member 120 may be two, or three or more. That is, the medical observation apparatus 100 includes two imaging devices, or three or more imaging devices. The imaging directions in the plurality of imaging devices may all be different, or some of the imaging directions may be the same. Furthermore, the imaging direction of each of the plurality of imaging devices may be fixed or may be changeable.

The configuration used for various known electronic imaging microscope units can be applied to the imaging device constituting the imaging member 120.

As an example, the imaging device that constitutes the imaging member 120 includes an optical system 120a and an image sensor 120b that includes an imaging element that captures an image of an observation target using light that has passed through the optical system 120a, for example. The optical system 120a includes optical elements such as one or two or more lenses including an objective lens, a zoom lens, and a focus lens, and a mirror. Examples of the image sensor 120b include an image sensor using a plurality of imaging elements, such as complementary metal oxide semiconductor (CMOS) and a charge coupled device (CCD).

The imaging member 120 having two or more imaging devices including the optical system 120a and the image sensor 120b functions as a stereo camera.

The imaging device constituting the imaging member 120 includes one or two or more functions such as a zoom function (one or both of an optical zoom function and an electronic zoom function) and an auto focus (AF) function, which are typically provided in an electronic imaging microscope unit.

The imaging device that constitutes the imaging member 120 may have a configuration capable of a so-called high resolution imaging, such as 4K or 8K. With a configuration in which the imaging device constituting the imaging member 120 is capable of high resolution imaging, it is possible to display an image on the display device 200 having a large display screen of 50 inches or more, for example, while ensuring a predetermined resolution (full HD image quality, for example). This improves the visibility of the practitioner as a viewer of the display screen of the display device 200. Furthermore, even when the medical image is enlarged by the electronic zoom function and displayed on the display screen of the display device 200, the imaging device included in the imaging member 120 is capable of imaging at a high resolution, making it possible to ensure a predetermined resolution. Furthermore, in a case where a predetermined resolution is ensured by using the electronic zoom function, the performance of the optical zoom function in the imaging device unit 106 can be suppressed. Accordingly, it is possible to simplify the optical system of the imaging device unit 106, leading to downsizing of the imaging device unit 106.

The imaging device unit 106 includes various operation devices for controlling the operation of the imaging device unit 106, for example. For example, the imaging device unit 106 in FIG. 3 includes a zoom switch 124, a focus switch 126, and an operation mode change switch 128. Needless to say, the positions and shapes of the zoom switch 124, the focus switch 126, and the operation mode change switch 128 are not limited to the example illustrated in FIG. 3.

The zoom switch 124 and the focus switch 126 are examples of operation devices for adjusting imaging conditions in the imaging device unit 106.

The zoom switch 124 includes, for example, a zoom-in switch 124a for increasing the zoom magnification (magnification magnification) and a zoom-out switch 124b for decreasing the zoom magnification. Operation on the zoom switch 124 can adjust zoom magnification, leading to zoom adjustment.

The focus switch 126 includes, for example, a distant view focus switch 126a that increases the focal length to the observation target (subject) and a near view focus switch 126b that decreases the focal length to the observation target. Operation on the focus switch 126 can adjust the focal length, leading to focus adjustment.

The operation mode change switch 128 is an example of an operation device for changing the operation mode of the arm 104 in the imaging device unit 106. Operation on the operation mode change switch 128 changes the operation mode of the arm 104. The operation modes of the arm 104 include, for example, the fixed mode and the free mode as described above.

An example of operation on the operation mode change switch 128 is operation of pressing the operation mode change switch 128. For example, while the practitioner presses the operation mode change switch 128, the operation mode of the arm 104 is set to the free mode. When the practitioner is not pressing the operation mode change switch 128, the operation mode of the arm 104 is set to the fixed mode.

Furthermore, the imaging device unit 106 includes a non-slip member 130 and a projection member 132 in order to further enhance operability and convenience when an operator performs operation on various operation devices.

The non-slip member 130 is a member provided to prevent the operation body from slipping when the operator operates the tubular member 122 with an operation tool such as a hand. The non-slip member 130 is formed of a material having a large friction coefficient, for example, and has a slip-resistant structure that is a member with irregularities.

The projection member 132 is a member provided to prevent a situation in which an operation tool such as a hand blocks the visual field of the optical system 120a when the operator operates the tubular member 122 with the operating tool or situation in which contamination of a cover glass slip (not illustrated) when the operation tool comes in contact with the cover glass slip in performing operation with the operating tool.

Needless to say, the position and the shape of each of the non-slip member 130 and the projection member 132 are not limited to the example illustrated in FIG. 3. Furthermore, it is allowable that the imaging device unit 106 omits one or both of the non-slip member 130 and the projection member 132.

An image signal (image data) generated by imaging in the imaging device unit 106 is transmitted to a processor functioning as a control unit described below, for example, and image processing is performed by the processor. The image processing according to the present embodiment corresponds to one or two or more of various processes such as gamma correction, white balance adjustment, enlargement or reduction of an image associated with the electronic zoom function, or pixel-to-pixel correction. In a case where the medical observation system according to the present embodiment includes a medical control apparatus (not illustrated) that controls various operations in the medical observation apparatus 100, the image processing according to the present embodiment may be performed on the medical control apparatus (not illustrated).

The medical observation apparatus 100 transmits a display control signal and an image signal that has undergone the above-described image processing to the display device 200.

Transmission of the display control signal and the image signal to the display device 200 allows a medical image obtained by capturing an observation target (for example, a captured image in which the surgical site is captured) to be displayed on the display screen of the display device 200 in a state of being enlarged or reduced to a desired magnification by one or both of the optical zoom function and the electronic zoom function.

Note that the configuration of the imaging device unit 106 is not limited to the above example.

For example, the imaging device unit 106 may include an adjustment mechanism that adjusts an inward angle (described below). An example of the adjustment mechanism will be described below.

The medical observation apparatus 100 has a hardware configuration illustrated with reference to FIGS. 2 and 3.

Note that the hardware configuration of the medical observation apparatus according to the present embodiment is not limited to the configuration illustrated with reference to FIGS. 2 and 3.

For example, the medical observation apparatus according to the present embodiment may have a configuration without the base 102 and capable of mounting the arm 104 directly onto a ceiling or a wall surface of the surgical room or the like. For example, in a case where the arm 104 is attached to the ceiling, the medical observation apparatus according to the present embodiment would have a configuration in which the arm 104 is hung from the ceiling.

FIG. 2 illustrates an example in which the arm 104 achieves six degrees of freedom concerning driving the imaging device unit 106. However, the configuration of the arm 104 is not limited to the configuration in which the freedom concerning the driving of the imaging device unit 106 is six degrees of freedom. For example, it is sufficient as long as the arm 104 is capable of appropriately moving the imaging device unit 106 according to the application. The number and arrangement of the joints and the links, the direction of the drive shaft of the joints, or the like can be appropriately set so that the arm 104 can have the desired degree of freedom.

FIGS. 2 and 3 illustrate examples in which various operation devices for controlling the operation of the imaging device unit 106 are provided in the imaging device unit 106. Some or all of the operation devices illustrated in FIGS. 2 and 3 may be omitted from the imaging device unit 106. For example, various operation devices for controlling the operation of the imaging device unit 106 may be provided in a site other than the imaging device unit 106 included in the medical observation apparatus according to the present embodiment. Furthermore, as another example, various operation devices for controlling the operation of the imaging device unit 106 may be external operation devices such as a foot switch and a remote controller.

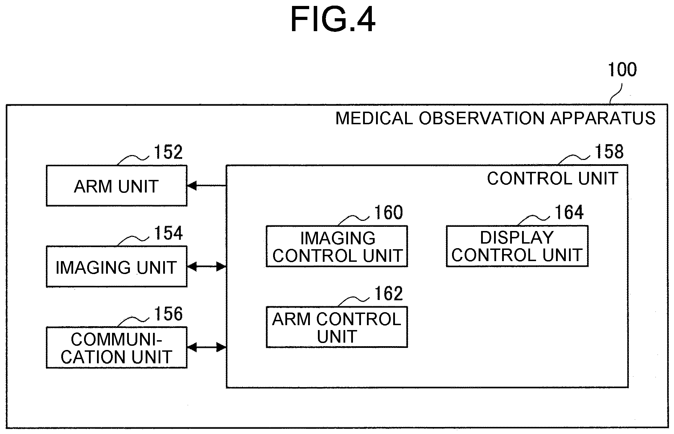

Next, the medical observation apparatus 100 illustrated in FIG. 2 will be described using functional blocks. FIG. 4 is a functional block diagram illustrating an example of a configuration of the medical observation apparatus 100 according to the present embodiment.

The medical observation apparatus 100 includes, for example, an arm unit 152, an imaging unit 154, a communication unit 156, and a control unit 158.

The arm unit 152 includes the arm 104 and supports the imaging device unit 106 included in the imaging unit 154.

The imaging unit 154 includes the imaging device unit 106 and images an observation target. The imaging on the imaging unit 154 is controlled by the control unit 158, for example.

The communication unit 156 is a communication unit included in the medical observation apparatus 100 and has a role of performing wireless or wired communication with an external device such as the display device 200. The communication unit 156 includes the above-described communication device (not illustrated), for example. Communication in the communication unit 156 is controlled by the control unit 158, for example.

The control unit 158 includes the above-described processor (not illustrated), for example, and has a role of controlling the entire medical observation apparatus 100. Furthermore, the control unit 158 plays a leading role in performing a process related to a control method described below. Note that the process related to the control method in the control unit 158 may be performed in a distributed manner by a plurality of processing circuits (for example, a plurality of processors).

More specifically, the control unit 158 includes, for example, an imaging control unit 160, an arm control unit 162, and a display control unit 164.

The imaging control unit 160 has a role of performing an imaging control process according to a control method described below and controls the imaging device unit 106 included in the imaging unit 154. The control in the imaging control unit 160 includes, for example, "control to achieve a match between an inward angle (described below) and a convergence angle (described below)" according to the control method described below.

In addition, concerning the control of the imaging device unit 106, the imaging control unit 160 can control one or two or more functions provided in an ordinary electronic microscope unit, such as control of a zoom function (one or both of an optical zoom function and an electronic zoom function) and control of an AF function, for example.

The arm control unit 162 controls driving of the arm 104 included in the arm unit 152. An example of the drive control of the arm 104 includes "applying a control signal for controlling the drive to the actuators (not illustrated) corresponding to each of the "joints 110a, 110b, 110c, 110d, 110e, and 110f".

The display control unit 164 has a role of performing a display control process according to a control method described below and controls display of a medical image on the display device 200.

The display control unit 164 transmits a display control signal and an image signal to a communication device (not illustrated) included in the communication unit 156, for example, so as to transmit the display control signal and the image signal to the display device 200, thereby controls the display on the display device 200. Note that communication control in the communication unit 156 may be performed by a communication control unit (not illustrated) included in the control unit 158.

The control unit 158 includes the imaging control unit 160 and the display control unit 164, for example, and thereby plays a leading role in performing processing according to the control method of the present embodiment. In addition, the control unit 158 includes the imaging control unit 160, the arm control unit 162, and the display control unit 164, for example, and thereby plays a roll of controlling the entire medical observation apparatus 100.

Note that the configuration of the control unit 158 is not limited to the example illustrated in FIG. 4.

For example, the control unit 158 can have an arbitrary configuration according to the method of dividing the functions of the medical observation apparatus 100, such as a configuration according to the method of dividing the process according to the control method of the present embodiment.

The medical observation apparatus 100 performs a process according to a control method of the present embodiment, which will be described below, with the configuration illustrated in FIG. 4, for example.

Note that the configuration of the medical observation apparatus according to the present embodiment is not limited to the configuration illustrated in FIG. 4.

For example, the medical observation apparatus according to the present embodiment can implement a part or all of the imaging control unit 160, the arm control unit 162, and the display control unit 164 illustrated in FIG. 4 separately from the control unit 158 (for example, using another processing circuit).

Furthermore, the configuration for implementing the process according to the control method of the present embodiment in the medical observation apparatus according to the present embodiment is not limited to the configuration illustrated in FIG. 4. For example, the medical observation apparatus according to the present embodiment can have a configuration according to the method of dividing the process according to the control method of the present embodiment.

Furthermore, for example, in a case of performing communication with an external device via an external communication device having a function and configuration similar to those of the communication unit 156, the medical observation apparatus according to the present embodiment may omit the communication unit 156.

Furthermore, in a case where the medical observation system according to the present embodiment has a configuration including a medical control apparatus (not illustrated) and the medical observation apparatus according to the present embodiment is controlled by the medical control apparatus (not illustrated), the medical observation apparatus according to the present embodiment may omit the control unit 158.

Here, the medical control apparatus (not illustrated) includes a control unit having the function and configuration similar to those of the control unit 158, and thereby performs a process according to a control method of the present embodiment described below and controls the operation of individual components such as the arm unit 152 and the imaging unit 154 included in the medical observation apparatus according to the present embodiment. The medical control apparatus (not illustrated) communicates with the medical observation apparatus according to the present embodiment via an internal communication device, or an external communication device connected to the apparatus, and thereby controls operation of individual components included in the medical observation apparatus according to the present embodiment.

Furthermore, in a case where the medical observation system according to the present embodiment has a configuration including a medical control apparatus (not illustrated) and the medical observation apparatus according to the present embodiment is controlled by the medical control apparatus (not illustrated), the medical observation apparatus according to the present embodiment may partially omit the functions of the control unit 158.

### [2] Control method according to the present embodiment

Next, processes according to the control method of the present embodiment will be described. Hereinafter, the processes according to the control method of the present embodiment will be described using an exemplary case where the processes are performed by the medical observation apparatus 100 (more specifically, the control unit 158 included in the medical observation apparatus 100, for example). As described above, in the medical observation system according to the present embodiment, the processes according to the control method of the present embodiment may be performed by a medical control apparatus (not illustrated).

### [2-1] Outline of control method according to the present embodiment

As described above, in a case where a plurality of display devices is arranged at the surgical operation field, the convergence angle varies for each of display devices due to various factors such as the location of the display device and the size of the display screen. Furthermore, along with the convergence angle changing for each of display devices, the inward angle for reproducing a natural three-dimensional effect with less fatigue also changes for each of the display devices.

The convergence angle according to the present embodiment is "an angle formed between the right eye and the left eye of a person viewing a display screen on which a medical image for the right eye and a medical image for the left eye are displayed". The convergence angle corresponds to "an angle formed between the line of sight of the right eye and the line of sight of the left eye when a certain position on the display screen is in focus", for example. The convergence angle corresponding to the display device 200 can be estimated from a representative value (for example, an average value) of human's interocular distance and a set value of the distance between the viewer of the display screen and the display screen (hereinafter, sometimes referred to as "viewing distance". Hereinafter, a case where the convergence angle corresponding to the display device 200 is set in advance for each of display devices will be described as an example. Note that the convergence angle corresponding to the display device 200 may be set to a value corresponding to a specific person on the basis of an interocular distance value set for a specific person and a detection result of a distance (viewing distance) between the specific person and the display screen detected by a distance sensor or the like.

The inward angle according to the present embodiment is "an angle formed by an imaging direction in an imaging device that captures a medical image for the right eye and an imaging direction in an imaging device that captures a medical image for the left eye". Hereinafter, an imaging device that captures the medical image for the right eye is referred to as a "first imaging device", and an imaging device that captures the medical image for the left eye is referred to as a "second imaging device".

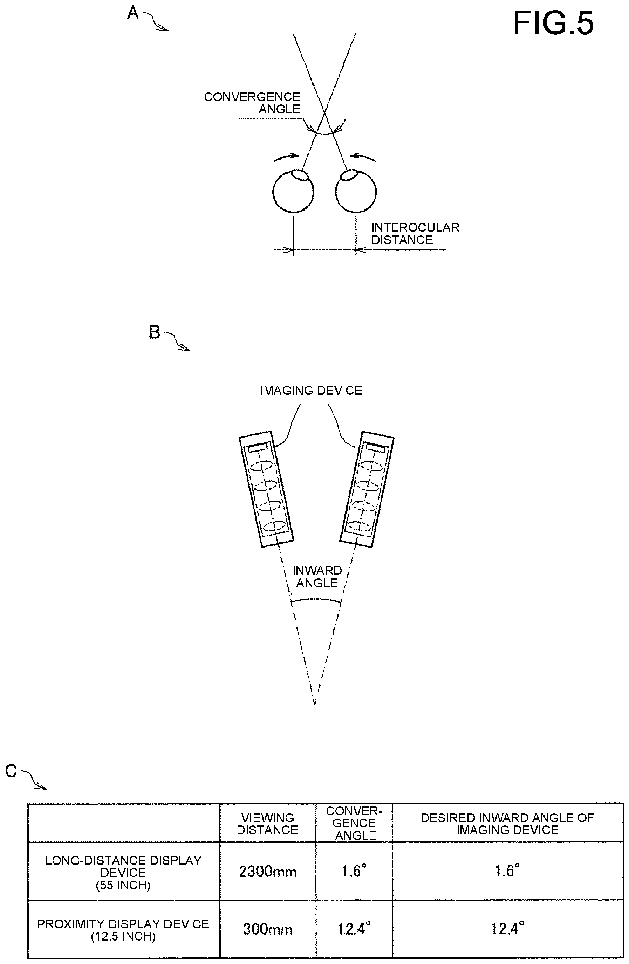

FIG. 5 is a view illustrating the control method according to the present embodiment. A of FIG. 5 conceptually illustrates a convergence angle, and B of FIG. 5 conceptually illustrates an inward angle. C of FIG. 5 illustrates the relationship between the display device size, the viewing distance, the convergence angle, and the desired inward angle in a case where the interocular distance is assumed to be 65 [mm].

As described above, the greater the difference between the inward angle and the convergence angle, the more the viewer of the medical image displayed on the display screen feels that the image is a flat image with no stereoscopic effect or that the image is a distorted image with an excessive three-dimensional effect. More specifically, the greater the difference between the inward angle and the convergence angle, the more the perception in the depth direction changes. In addition, the greater the viewing distance, the greater the sense of depth perceived.

Therefore, it is desirable to achieve a match between the inward angle and the convergence angle in order to give a more natural three-dimensional effect to the viewer of the medical image displayed on the display screen of the display device 200. When a match between the inward angle and the convergence angle is achieved, it is possible to achieve a more precise view with respect to the horizontal direction and the depth direction, enabling acquisition of a more natural three-dimensional effect.

Therefore, the medical observation apparatus 100 controls the imaging in the imaging device included in the imaging device unit106 such that the inward angle is adjusted corresponding to a display device such as the display device 200 that displays the medical image for the right eye and the medical image for the left eye. That is, the medical observation apparatus 100 adjusts the inward angle to the inward angle corresponding to the display device that displays the medical image for the right eye and the medical image for the left eye.

The medical observation apparatus 100 adjusts the inward angle on the basis of the convergence angle. More specifically, the medical observation apparatus 100 changes a baseline length between the first imaging device and the second imaging device and thereby adjusts the inward angle to the convergence angle corresponding to the display device 200 that displays the medical image for the right eye and the medical image for the left eye on the display screen. Hereinafter, the medical image for the right eye and the medical image for the left eye will be collectively referred to as a "stereoscopic image" or simply as a "medical image" in some cases.

As described above, adjusting the inward angle to the convergence angle corresponding to the display device 200 that displays the medical image enables the medical observation apparatus 100 to obtain a medical image that can give a more natural three-dimensional effect. In other words, the medical observation apparatus 100 adjusts the inward angle to the convergence angle corresponding to the display device 200 that displays the medical image, and thereby further optimizes imaging of each of the medical image for the right eye and the medical image for the left eye, that is, the imaging for obtaining a stereoscopic image.

Acquisition of a medical image that gives a more natural three-dimensional effect enables a viewer of the display screen on which the medical image is displayed to feel a more natural three-dimensional effect. Furthermore, feeling a more natural three-dimensional effect leads to a reduction in fatigue when viewing a stereoscopic image.

### [2-2] Processing according to control method of the present embodiment

Next, the processes according to the control method of the present embodiment will be described more specifically.

The medical observation apparatus 100 performs, for example, an imaging control process illustrated in the following (1) and a display control process illustrated in the following (2) as processes according to the control method of the present embodiment.

Note that the processing according to the control method of the present embodiment is not limited to the example described above. For example, the medical observation apparatus 100 need not perform the display control process illustrated in (2) below and may allow the display control process to be performed by an external device. Even in a case where the medical observation apparatus 100 does not perform the display control process illustrated in (2) below, the medical observation apparatus 100 can obtain a medical image with a more natural three-dimensional effect.

Furthermore, adjustment of the inward angle to the convergence angle corresponding to the display device 200 that displays the medical image can also be achieved by mounting an adapter (that is, an external device of the medical observation apparatus 100) on the imaging device unit 106, for example. In a case where the inward angle is adjusted to the convergence angle corresponding to the display device 200 by the adapter, the medical observation apparatus 100 need not perform the imaging control process illustrated in (1) below.

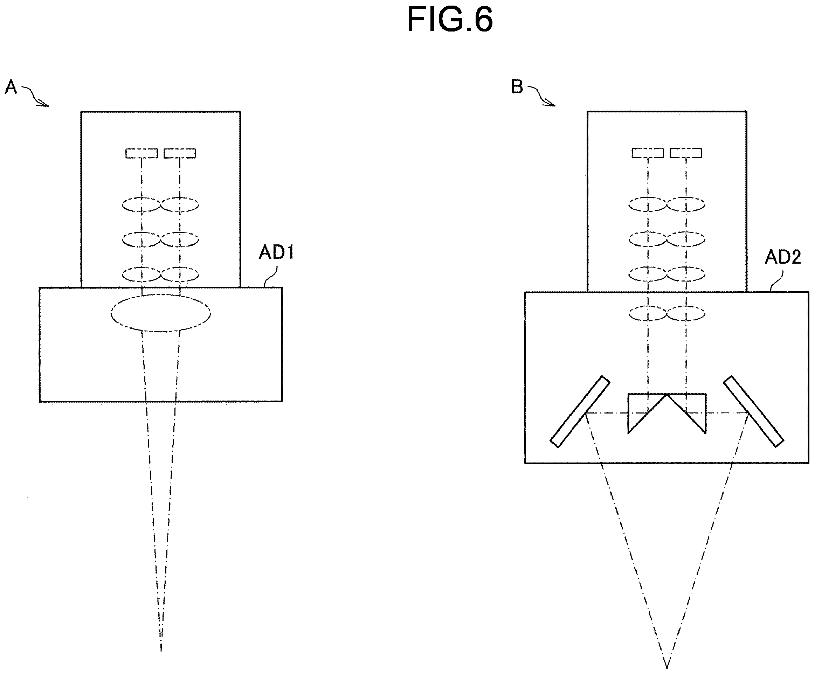

FIG. 6 is a view illustrating an example of an adapter according to the present embodiment that can adjust an inward angle to a convergence angle corresponding to the display device 200. An adapter AD1 illustrated in A of FIG. 6 illustrates an example of an adapter corresponding to a display device 200A illustrated in FIG. 1. An adapter AD2 illustrated in B of FIG. 6 illustrates an example of an adapter corresponding to a display device 200B illustrated in FIG. 1. Needless to say, examples of the adapter according to the present embodiment are not limited to the example illustrated in FIG. 6.

For example, mounting an adapter corresponding to each of the display devices 200 as illustrated in FIG. 6 on the imaging device unit 106 enables a viewer of the display screen of the display device 200 corresponding to the mounted adapter to feel a more natural three-dimensional effect.

### (1) Imaging control process

As described above, the medical observation apparatus 100 adjusts the inward angle to the inward angle corresponding to the display device 200 that displays a medical image (an example of a display device that displays a medical image for the right eye and a medical image for the left eye). More specifically, the medical observation apparatus 100 changes the baseline length between the first imaging device and the second imaging device, and thereby adjusts the inward angle to the convergence angle corresponding to the display device 200 that displays a medical image. The imaging control process is performed by the imaging control unit 160 illustrated in FIG. 4, for example.

In other words, the medical observation apparatus 100 sets the convergence angle corresponding to the display device 200 that displays the medical image for the right eye and the medical image for the left eye and adjusts the inward angle to achieve a match with the set convergence angle.

The medical observation apparatus 100 refers to "a table (or a database, similar applies hereinafter) associating data specifying the display device 200 (for example, ID of the display device 200) stored in a recording medium that functions as a storage unit (not illustrated) with data indicating a convergence angle", for example, and thereby sets the convergence angle corresponding to the display device 200 that displays the medical image. Setting of the display device 200 that displays a medical image is performed at an arbitrary timing such as a timing of installation of the display device 200 by operation on an operation device such as a remote controller. Registration of data to the table and alteration and deletion of various types of data stored in the table can be appropriately performed by operation on an operation device such as a remote controller.

Note that the method of setting the convergence angle corresponding to the display device 200 is not limited to the above example. For example, the medical observation apparatus 100 may use an arbitrary algorithm that can uniquely determine a convergence angle on the basis of data specifying the display device 200 that displays a medical image and may thereby set a convergence angle corresponding to the display device 200 that displays a medical image.

The processes of adjusting the inward angle to the convergence angle corresponding to the display device 200 that displays the medical image include an imaging control process according to a first example illustrated in (1-1) below, and an imaging control process according to a second example illustrated in (1-2) below.

### (1-1) First example of imaging control processing

The medical observation apparatus 100 controls the adjustment mechanism for adjusting the inward angle to adjust the inward angle and thereby achieves a match between the inward angle and the convergence angle. As described above, the medical observation apparatus 100 adjusts the inward angle to correspond to the display device 200.

The adjustment mechanism is provided in the imaging device unit 106, for example, as described above.

Examples of the adjustment mechanism include a movement mechanism (hereinafter, referred to as a "first movement mechanism") capable of moving one or both of the first imaging device and the second imaging device. The first movement mechanism drives one or both of a motor that moves the first imaging device and a motor that moves the second imaging device to move one or both of the first imaging device and the second imaging device. Note that the first movement mechanism is not limited to the example described above and may have any configuration capable of moving the imaging device.

The medical observation apparatus 100 controls the movement of one or both of the first imaging device and the second imaging device performed by the first movement mechanism and thereby adjusts the inward angle. The medical observation apparatus 100 outputs a drive control signal for controlling the drive of the first movement mechanism to the first movement mechanism, for example, and thereby adjusts the inward angle. An example of the drive control signal is a signal of any format for controlling the rotation direction and the rotation amount of each of the motors included in the first movement mechanism.

Another example of the adjustment mechanism is a movement mechanism (hereinafter referred to as a second movement mechanism) that can change one or both of the imaging direction of the first imaging device and the imaging direction of the second imaging device by moving the optical system. The second movement mechanism includes a mirror (an example of an optical system) corresponding to each of the first imaging device and the second imaging device, and a motor that moves each of the mirrors, for example. The second movement mechanism moves one or both of the imaging direction of the first imaging device and the imaging direction of the second imaging device by driving each of the motors corresponding to the mirror. The second movement mechanism is not limited to the example described above. For example, the second movement mechanism may have a "configuration including a prism (another example of an optical system) corresponding to each of the first imaging device and the second imaging device, and a motor that moves each of the prisms"

The medical observation apparatus 100 controls the movement of the optical system performed by the second movement mechanism and thereby adjusts the inward angle. The medical observation apparatus 100 outputs a drive control signal for controlling the driving of the second movement mechanism to the second movement mechanism and thereby adjusts the inward angle, for example. An example of the drive control signal is a signal of any format for controlling the rotation direction and the rotation amount of each of the motors included in the second movement mechanism.

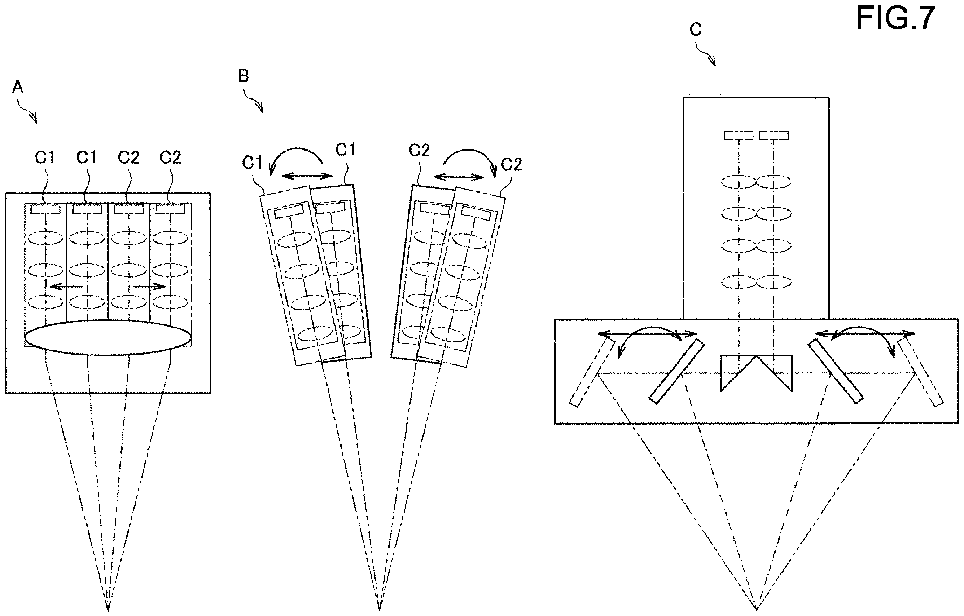

FIG. 7 is a view illustrating an example of a configuration of the adjustment mechanism according to the present embodiment. A of FIG. 7 illustrates an example of the first movement mechanism, that is, an example of a "zoom system shift-type adjustment mechanism that moves one or both of a first imaging device C1 and a second imaging device C2 with respect to a zoom lens shared by the first imaging device C1 and the second imaging device C2 and thereby adjusts the inward angle". B of FIG. 7 illustrates another example of the first movement mechanism, that is, an example of an "adjustment mechanism that changes the position of one or both of the first imaging device C1 and the second imaging device C2 and thereby adjusts the inward angle". C of FIG. 7 illustrates an example of the second movement mechanism, that is, an example of "a mirror moving adjustment mechanism that adjusts an inward angle by moving a mirror".

Needless to say, examples of the configuration of the adjustment mechanism are not limited to the example illustrated in FIG. 7.

### (1-2) Second example of imaging control process

As described above, the medical observation apparatus 100 has a configuration including three or more imaging devices having different imaging directions in some cases. In this case, the medical observation apparatus 100 selects the first imaging device and the second imaging device from three or more imaging devices having different imaging directions and thereby achieves a match between the inward angle and the convergence angle. The medical observation apparatus 100 selects the first imaging device and the second imaging device so as to correspond to the display device 200 and thereby adjusts the inward angle so as to correspond to the display device 200.

In the imaging control process according to the second example, the inward angle is adjusted by selecting the first imaging device and the second imaging device. Therefore, the imaging device unit 106 does not need to include an adjustment mechanism. That is, the imaging control process according to the second example can adjust the inward angle in the medical observation apparatus 100 in which the imaging device unit 106 includes no adjustment mechanism. Note that the imaging control process according to the second example can also be applied to the medical observation apparatus 100 in which the imaging device unit 106 includes an adjustment mechanism.

The medical observation apparatus 100 selects a first imaging device and a second imaging device from three or more imaging devices arranged on a same plane.

Examples in which the medical observation apparatus 100 selects the first imaging device and the second imaging device include a first selection example illustrated in the following (a), and a second selection example illustrated in the following (b). Needless to say, examples in which the medical observation apparatus 100 selects the first imaging device and the second imaging device are not limited to the first selection example illustrated in (a) below and the second selection example illustrated in (b) below.

### (a) First selection example

For example, in a case where three or more imaging devices are arranged on one plane, the medical observation apparatus 100 selects two imaging devices from the three or more imaging devices arranged on the plane. The two selected imaging devices will be the first imaging device and the second imaging device.

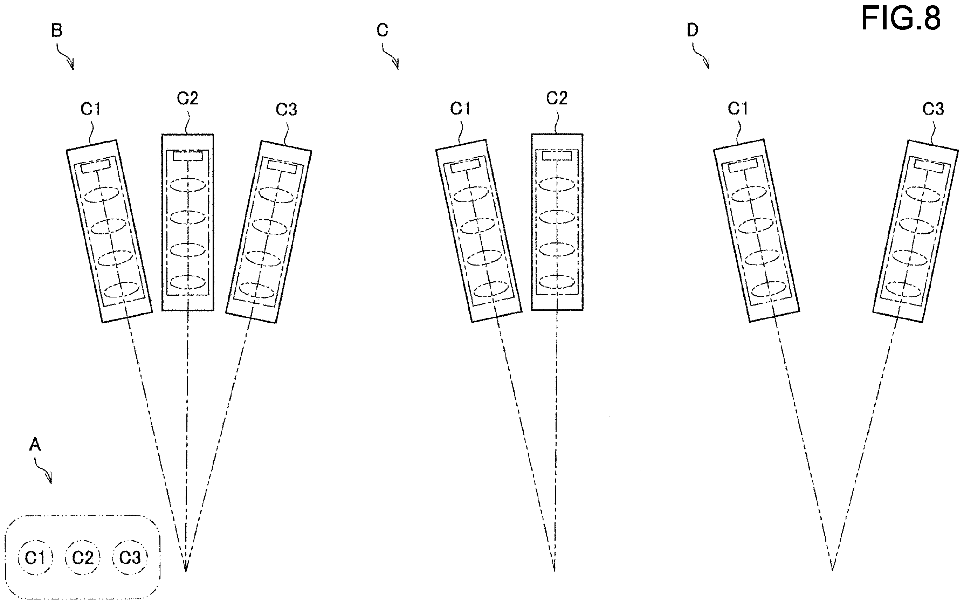

FIG. 8 is a view illustrating an example of an imaging control process according to the control method of the present embodiment, illustrating an example of imaging control process according to a second example in a case where the imaging device unit 106 includes three imaging devices C1, C2, and C3. A of FIG. 8 conceptually illustrates arrangement of the imaging devices C1, C2, and C3, that is, "arrangement of the imaging devices C1, C2, and C3 when viewed from the aperture surface side of the lower end (an end of lower side in FIG. 3) of the tubular member 122 illustrated in FIG. 3". B of FIG. 8 illustrates a state before the first imaging device and the second imaging device are selected from the imaging devices C1, C2, and C3. Each of C of FIG. 8 and D of FIG. 8 illustrates a state after the first imaging device and the second imaging device are selected from the imaging devices C1, C2, and C3.

As illustrated in C of FIG. 8 and D of FIG. 8, the inward angle varies depending on the method of selecting two imaging devices from the imaging devices C1, C2, and C3. Therefore, the medical observation apparatus 100 selects the first imaging device and the second imaging device from the three imaging devices C1, C2, and C3 arranged on the same plane, thereby achieving a match between the inward angle and convergence angle.

Note that, even in a case where the imaging device unit 106 has four or more imaging devices, and the four or more imaging devices are disposed on one plane, the medical observation apparatus 100 can achieve a match between the inward angle and the convergence angle similarly to the case described with reference to FIG. 8.

Furthermore, an example of selecting an imaging device in a case where the imaging device unit 106 has three or more imaging devices is not limited to the example illustrated in FIG. 8. For example, the medical observation apparatus 100 can select one imaging device from three or more imaging devices arranged on the same plane.

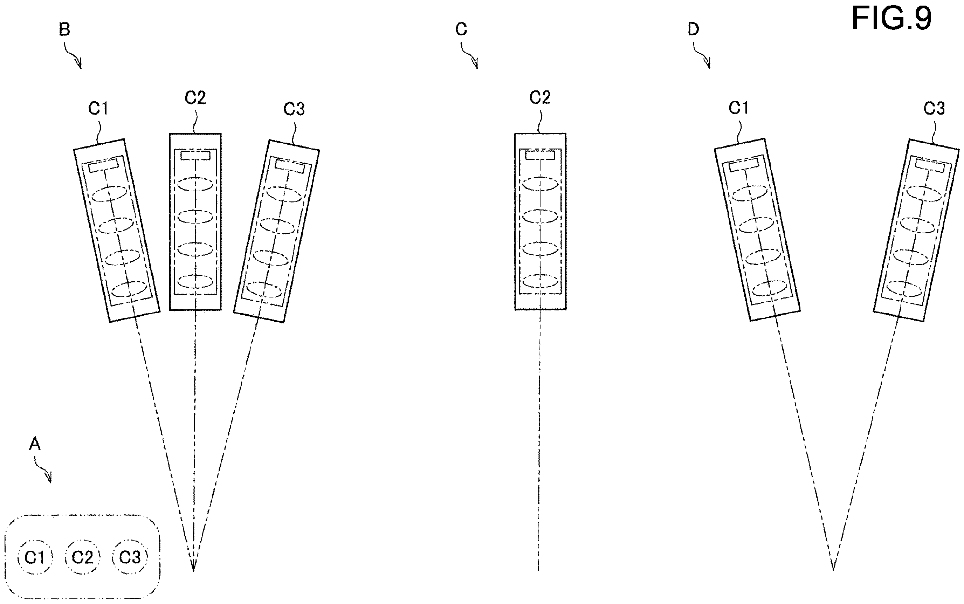

FIG. 9 is a view illustrating an example of an imaging control process according to the control method of the present embodiment, illustrating another example of selecting the imaging device in a case where the imaging device unit 106 includes three imaging devices C1, C2, and C3. A of FIG. 9 conceptually illustrates the arrangement of the imaging devices C1, C2, and C3, similarly to A of FIG. 8. B of FIG. 9 illustrates a state before an imaging device is selected from the imaging devices C1, C2, and C3. Each of C of FIG. 9 and D of FIG. 9 illustrates a state after an imaging device is selected from the imaging devices C1, C2, and C3.

As illustrated in C of FIG. 9 and D of FIG. 9, the medical observation apparatus 100 can select one or two imaging devices from three imaging devices C1, C2, and C3. As illustrated in C of FIG. 9 and D of FIG. 9, in a case where the medical observation apparatus 100 can switch the number of imaging devices to be selected, for example, it is possible to implement switching between imaging in wide angle view (overhead view) by one imaging device and imaging of a stereoscopic image by two imaging devices. Furthermore, at the time of imaging of the stereoscopic image using two imaging devices, enlargement by optical zoom or electronic zoom may be further performed.

The number of imaging devices selected by the medical observation apparatus 100 is switched by operation on an operation device such as a remote controller or a foot switch.

Note that even in a case where the imaging device unit 106 has four or more imaging devices, and the four or more imaging devices are disposed on one plane, the medical observation apparatus 100 can switch the number of imaging devices to be selected similarly to the case described with reference to FIG. 9.

### (b) Second selection example

An example of the arrangement of the plurality of imaging devices in the imaging device unit 106 is not limited to the example of arrangement on one plane as illustrated in FIGS. 8 and 9. For example, in the imaging device unit 106, a plurality of imaging devices may be arranged on each of a first plane and a second plane orthogonal to the first plane. That is, in the imaging device unit 106, a plurality of imaging devices can be arranged perpendicularly to each other.

Here, the significance (the significance of perpendicular arrangement) of arranging a plurality of imaging devices on each of the first plane and the second plane will be described.

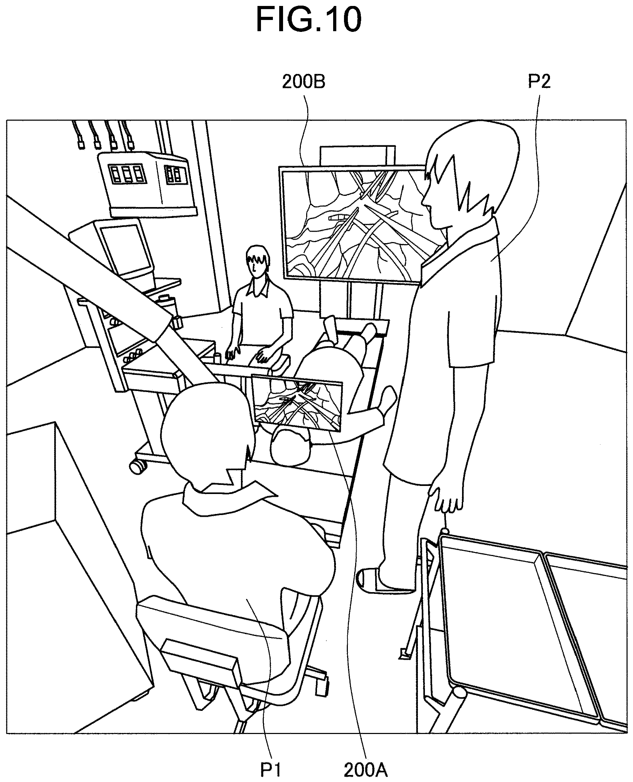

FIGS. 10 to 12 are views illustrating the significance of arranging a plurality of imaging devices on each of the first plane and the second plane according to the present embodiment.

FIG. 10 is a view mainly illustrating a surgical operation field where the medical observation system 1000 illustrated in FIG. 1 is used focusing on a practitioner P1 (an example of a medical worker). The practitioner P1 performs a medical practice while viewing a medical image displayed on the display device 200A, for example.

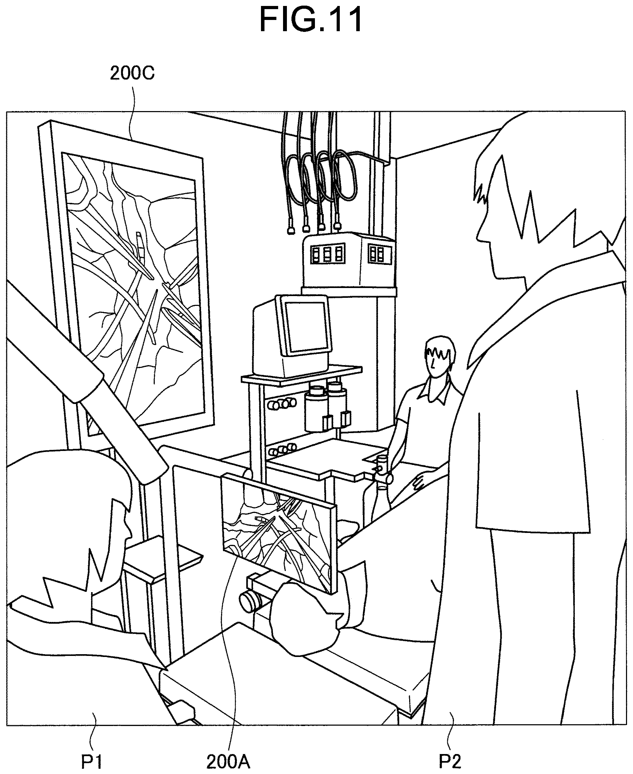

FIG. 11 is a view mainly illustrating a surgical operation field where the medical observation system 1000 illustrated in FIG. 1 is used focusing on an assistant P2 (another example of a medical worker). The assistant P2 performs a medical practice while viewing a medical image displayed on a display device 200C, for example.

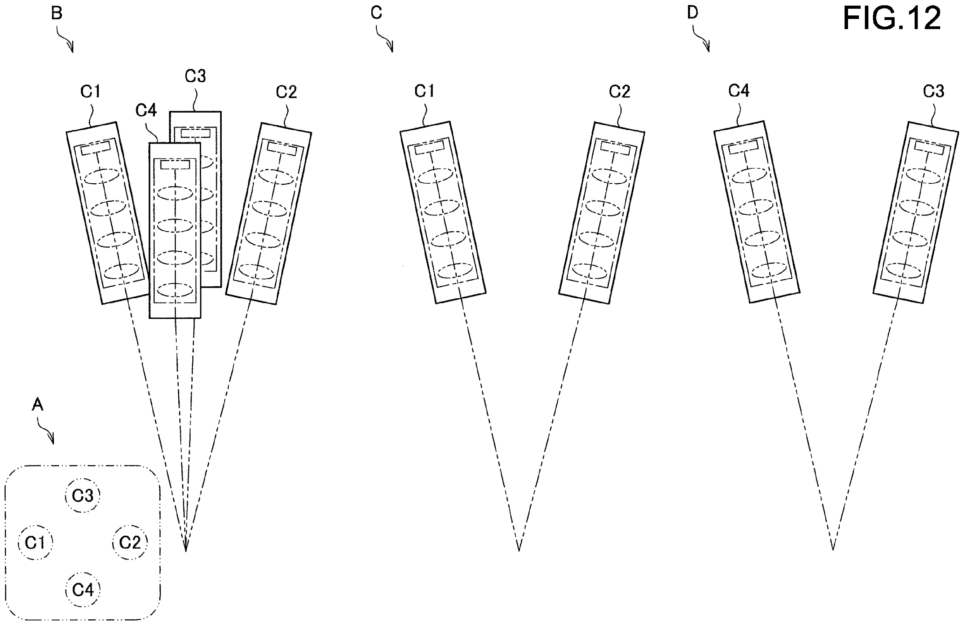

FIG. 12 illustrates an example of selecting an imaging device in a case where the imaging device unit 106 has four imaging devices C1, C2, C3, and C4. A of FIG. 12 conceptually illustrates the arrangement of the imaging devices C1, C2, C3, and C4, similarly to A in FIG. 8. In FIG. 12, the imaging devices C1 and C2 are arranged on the first plane (or on the second plane), and the imaging devices C3 and C4 are arranged on the second plane (or on the first plane). B of FIG. 12 illustrates a state before an imaging device is selected from the imaging devices C1, C2, C3, and C4. In addition, each of C in FIG. 12 and D in FIG. 12 illustrates a state after an imaging device is selected from the imaging devices C1, C2, C3, and C4.

For example, as illustrated in FIGS. 10 and 11, in a case where the assistant P2 performs a medical practice in a position perpendicular to the practitioner P1, the imaging direction of an imaging device that captures a medical image for the practitioner P1 is perpendicular to the front direction of the assistant P1. In this case, this makes it difficult to achieve communication using good hand-eye coordination between the assistant P2 and the practitioner P1. As one method for reducing the difficulty in achieving such communication using good hand-eye coordination, there is considered to, as illustrated in FIGS. 10 and 11, "set the aspect ratio of the display screen of the display device 200C for the assistant P2 opposite to the aspect ratio of the display screen of the display device 200A for the practitioner P1." However, simply setting the aspect ratio of the display screen of the display device 200C for the assistant P2 opposite to the aspect ratio of the display screen of the display device 200A for the practitioner P1 would not achieve display of the stereoscopic image on the display device 200C.

Therefore, in the medical observation apparatus 100, as illustrated in A of FIG. 12, the imaging devices C1 and C2 are disposed on the first plane (or on the second plane), and the imaging devices C3 and C4 are disposed on the second plane (or on the first plane).

In addition, as illustrated in C of FIG. 12, the medical observation apparatus 100 selects the imaging devices C1 and C2 as the imaging devices that capture the medical image to be displayed on the display screen of the display device 200A for the practitioner P1. Furthermore, as illustrated in E of FIG. 12, the medical observation apparatus 100 selects the imaging devices C3 and C4 as the imaging devices that capture the medical image to be displayed on the display screen of the display device 200C for the assistant P2.

For example, selecting the imaging devices as illustrated in FIG. 12 enables each of the practitioner P1 and the assistant P2 to view a stereoscopic image that has captured the same position.

Here, in a case where the four imaging devices C1, C2, C3, and C4 are arranged in the imaging device unit 106 as illustrated in FIG. 12 and the imaging device unit 106 includes an adjustment mechanism, it is possible to adjust the inward angle so as to correspond to the display device 200 by the imaging control process according to the first example described above.

However, in a case where the imaging device unit 106 does not include the adjustment mechanism, the inward angle cannot be adjusted by the imaging control process according to the first example described above.

Therefore, the medical observation apparatus 100 further performs the process according to the first selection example illustrated in (a) above to adjust the inward angle by the imaging control process according to the second example.

More specifically, in the imaging device unit 106 included in the medical observation apparatus 100 to which the process according to the first selection example is further applied, three or more imaging devices are arranged on the first plane, and three or more imaging devices are arranged on the second plane. Here, a part of the imaging device arranged on the first plane may be an imaging device arranged on the second plane. That is, the medical observation apparatus 100 to which the process according to the first selection example is further applied includes at least five imaging devices.

Then, the medical observation apparatus 100 selects the first imaging device and the second image device from three or more imaging devices arranged on the first plane, or three or more imaging devices arranged on the second plane.

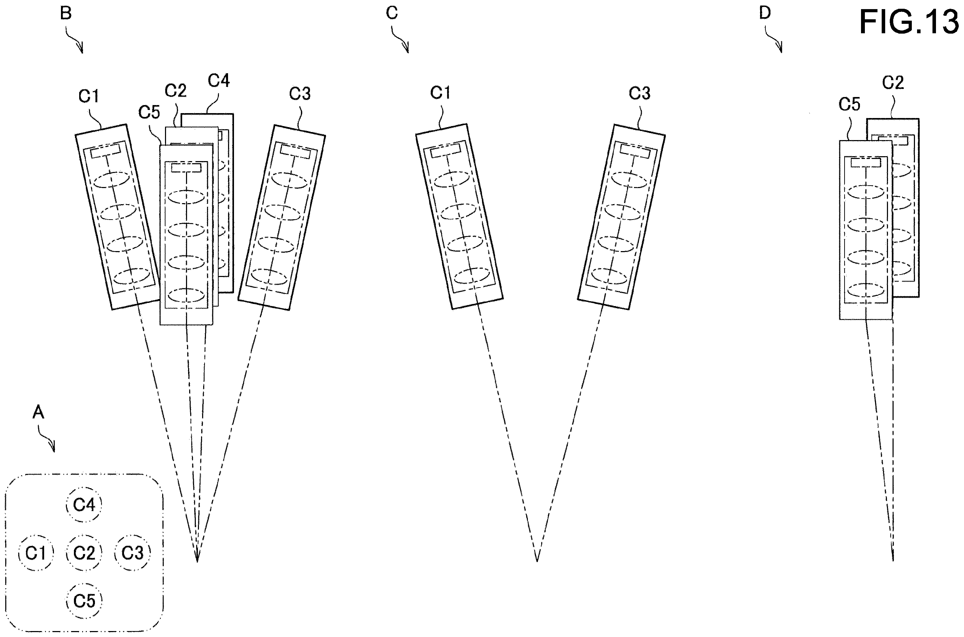

FIG. 13 is a view illustrating an example of an imaging control process according to the control method of the present embodiment, illustrating an example of selecting the imaging device in a case where the imaging device unit 106 includes five imaging devices C1, C2, C3, C4, and C5. A of FIG. 13 conceptually illustrates the arrangement of the imaging devices C1, C2, C3, C4, and C5, similarly to A of FIG. 8. B of FIG. 13 illustrates a state before an imaging device is selected from the imaging devices C1, C2, C3, C4, and C5. In addition, each of C of FIG. 13 and D of FIG. 13 illustrates a state after an imaging device is selected from the imaging devices C1, C2, C3, C4, and C5.

For example, as illustrated in C of FIG. 13, in a case of obtaining a medical image to be displayed on the display device 200A for the practitioner P1, two imaging devices are selected from the imaging devices C1, C2, and C3 arranged on the first plane (or the second plane) Moreover, as illustrated in D of FIG. 13, in a case of obtaining a medical image to be displayed on the display device 200C for the assistant P2, for example, two imaging devices are selected from the devices C4, C2, and C5 arranged on the second plane (or the first plane). For example, as illustrated in C of FIG. 13 and D of FIG. 13, two image devices are selected from three or more imaging devices arranged on the first plane, or from three or more imaging devices arranged on the second plane, whereby achieving a match between the inward angle and the convergence angle similarly to the case where the process according to the first selection example illustrated in (a) is performed.

Although FIG. 13 illustrates an example in which the imaging device unit 106 includes five imaging devices, it is needless to say that the number of imaging devices included in the imaging device unit 106 is not limited to five. Moreover, for example, as illustrated with reference to FIG. 9, the medical observation apparatus 100 is also capable of selecting one imaging device from three or more imaging devices arranged on the first plane or three or more imaging devices arranged on the second plane.

### (2) Display control process

The medical observation apparatus 100 controls to display a medical image (a medical image for the right eye and a medical image for the left eye) captured by the imaging device unit 106 on a display screen of a predetermined display device 200. The display control process is performed by the display control unit 164 illustrated in FIG. 4, for example.

The medical observation apparatus 100 transmits a display control signal and an image signal indicating a medical image to the predetermined display device 200 and thereby controls to display a medical image on the display screen of the predetermined display device 200, for example. Note that, as described above, the medical observation apparatus 100 may perform image processing on the medical image and may transmit the image signal that has undergone the image processing to the predetermined display device 200.

Examples of the predetermined display device 200 include the display device 200 corresponding to the adjustment of the inward angle in the first imaging control process illustrated in the above (1), or the display device 200 corresponding to the selection of the imaging device in the second imaging control process illustrated in the above (1) .

### (2-1) First example of display control process: example of display control process in a case where first imaging control process is performed

In a case where the first imaging control process illustrated in the above (1) is performed, the medical observation apparatus 100 controls to display the medical image captured after the inward angle has been adjusted (the medical image for the right eye and the medical image for the left eye) on the display screen of the predetermined display device 200, for example.

Note that the medical observation apparatus 100 can control to display a medical image captured before the inward angle is adjusted in the first imaging control process illustrated in above-described (1) on a display screen of a predetermined display device 200. Furthermore, the medical observation apparatus 100 can control to display a medical image captured while the inward angle is being adjusted in the first imaging control process illustrated in the above-described (1) on a display screen of a predetermined display device 200.

### (2-2) Second example of display control process: example of display control process in a case where second imaging control process is performed

In a case where the second imaging control process illustrated in the above-described (1) is performed, the medical observation apparatus 100 controls to display the medical image for the right eye captured by the selected first imaging device and the medical image for the left eye captured by the selected second medical imaging device on a display screen of a predetermined display device 200.

Note that the display control process according to the second example is not limited to the example described above.

For example, the medical observation apparatus 100 may selectively perform a rotation process of rotating the medical image for the right eye and the medical image for the left eye so as to correspond to the predetermined display device 200.

The medical observation apparatus 100 determines whether to perform the rotation process for each of the predetermined display devices 200, for example, and thereby selectively performs the rotation process. The medical observation apparatus 100 determines whether to perform the rotation process with reference to "a table (or a database) associating data specifying the display device 200 (for example, the ID of the display device 200)" stored in a recording medium that functions as a storage unit (not illustrated) with data (a flag, for example) indicating whether to perform the rotation process, for example. The medical observation apparatus 100 may determine whether to perform the rotation process by an arbitrary algorithm capable of determining whether to perform the rotation process on the basis of the data that specifies the display device 200 that displays the medical image.

Accordingly, the medical observation apparatus 100 controls to display the medical image for the right eye and the medical image for the left eye that have undergone selective rotation process on the display screen of the predetermined display device 200.

Furthermore, the medical observation apparatus 100 may selectively perform a clipping process of clipping a part of the medical image for the right eye or a part of the medical image for the left eye so as to correspond to the predetermined display device 200, for example.

The medical observation apparatus 100 determines whether to perform the clipping process for each of the predetermined display devices 200, for example, and thereby selectively performs the clipping process. The medical observation apparatus 100 determines whether to perform the clipping process with reference to "a table (or a database) associating data specifying the display device 200 (for example, the ID of the display device 200) stored in a recording medium that functions as a storage unit (not illustrated) with data (a flag, for example) indicating whether to perform the clipping process", for example. The medical observation apparatus 100 may determine whether to perform the clipping process by an arbitrary algorithm capable of determining whether to perform the clipping process on the basis of the data that specifies the display device 200 that displays the medical image.

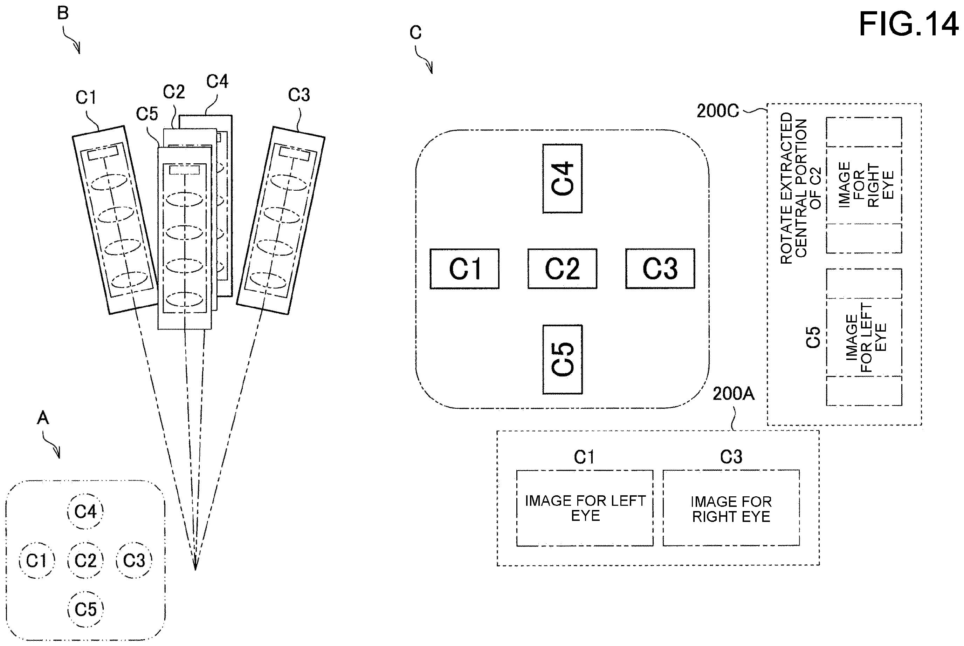

FIG. 14 is a view illustrating an example of the display control process according to the present embodiment. A of FIG. 14 conceptually illustrates arrangement of the imaging devices C1, C2, C3, C4, and C5, and the arrangement of the imaging devices C1, C2, C3, C4, and C5 is the same as A of FIG. 13. B of FIG. 14 illustrates a state before the imaging device is selected from the imaging devices C1, C2, C3, C4, and C5, and the state before the imaging device is selected is the same as B in FIG. 13. C of FIG. 14 conceptually illustrates a relationship between the imaging devices C1, C2, C3, C4, and C5, the medical image displayed on the display screen of the display device 200A for the practitioner P1, and the medical image displayed on the display screen of the display device 200C for the assistant P2. In FIG. 14, the medical image for the right eye is indicated as "image for right eye", and the medical image for the left eye is indicated as "image for left eye".

As illustrated in FIG. 14, the imaging device C2 is an imaging device arranged on the first plane, being an imaging device arranged on the second plane at the same time. Furthermore, as illustrated in C of FIG. 14, the horizontal axis of the imaging device C2 is orthogonal to the horizontal axis of each of the imaging devices C4 and C5. Therefore, in a case where the medical image captured by the imaging device C2 is directly displayed on the display screen of the display device 200C, the displayed medical image is not be recognized as a stereoscopic image. Therefore, in the case of displaying the medical image captured by the imaging device C2 on the display screen of the display device 200C, there is a need to perform the rotation process on the medical image captured by the imaging device C2 in order to achieve a match with respect to the medical image captured by the imaging devices C4 and C5.

In addition, a medical image captured by an imaging device is typically a horizontally long image (the number of pixels in the horizontal direction is larger than the number of pixels in the vertical direction). Therefore, even when rotation process is performed on the medical image captured by the imaging device C2, the displayed medical image might not be recognized as a stereoscopic image.

Therefore, in the example illustrated in FIG. 14, "in a case where the medical image captured by the imaging device C2 is displayed as a medical image for the right eye and the medical image captured by the imaging device C5 is displayed as a medical image for the left eye on the display screen of the display device 200C", the medical observation apparatus 100 performs the following process.
- Process on medical image for the right eye: performing "clipping process of clipping the central part of the medical image captured by the imaging device C2" and performing "rotation process of rotating the medical image by 90[°] to the right".
- Process on medical image for the left eye: performing "clipping process of clipping the same range as the clipping range of the medical image captured by the imaging device C2, from the medical image captured by the imaging device C5".

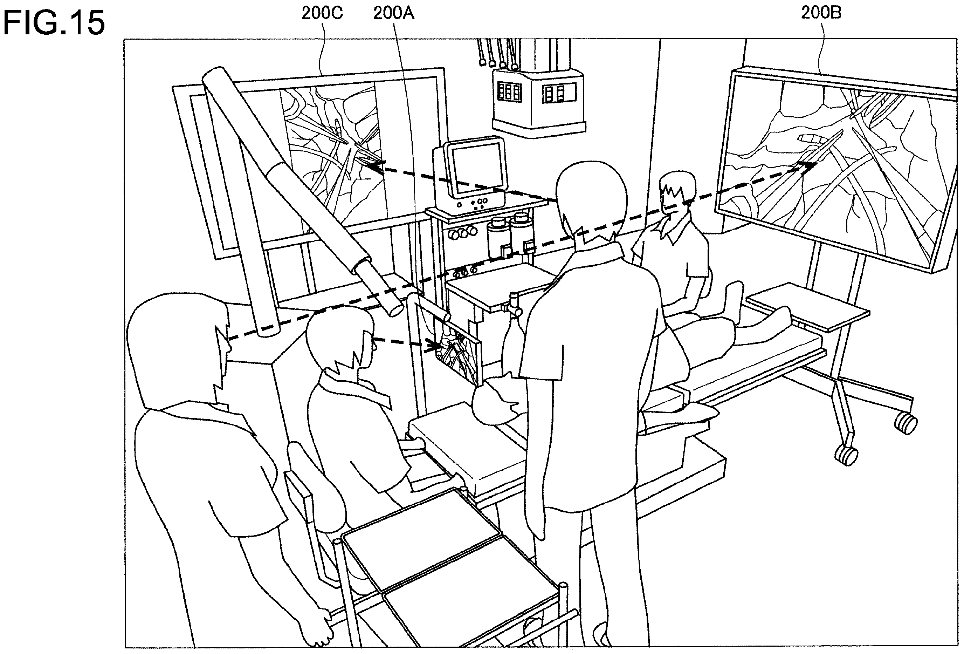

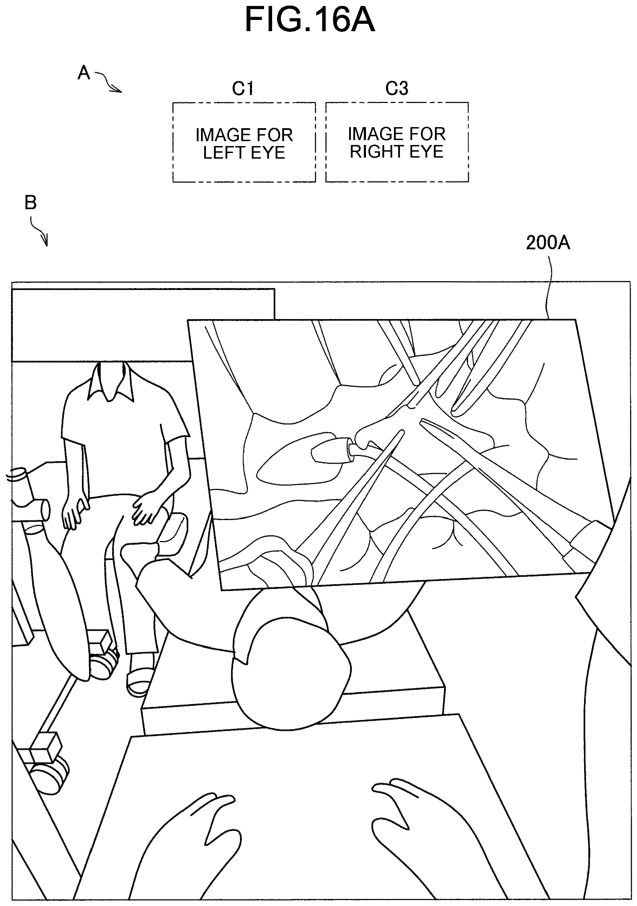

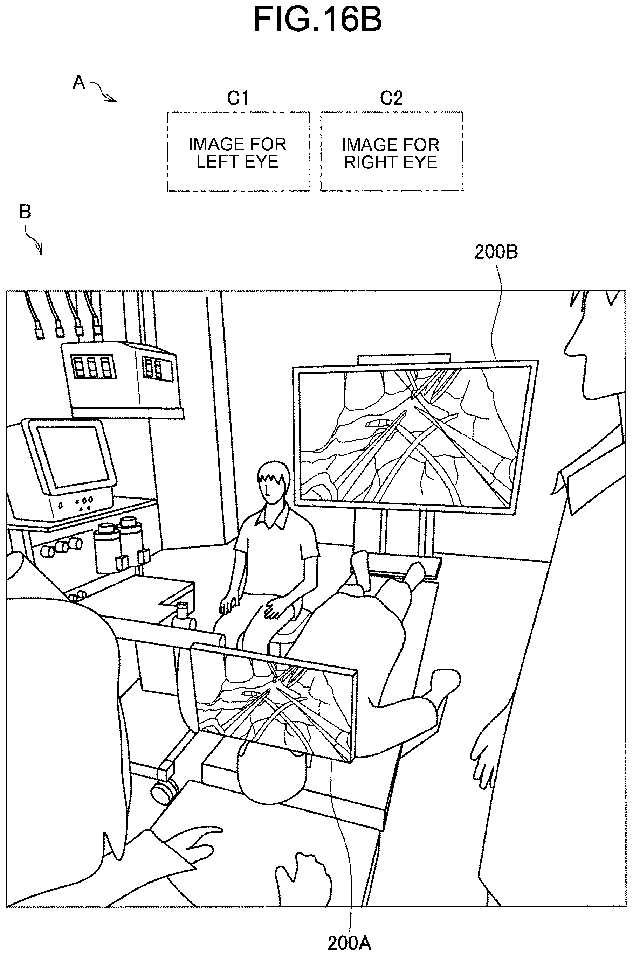

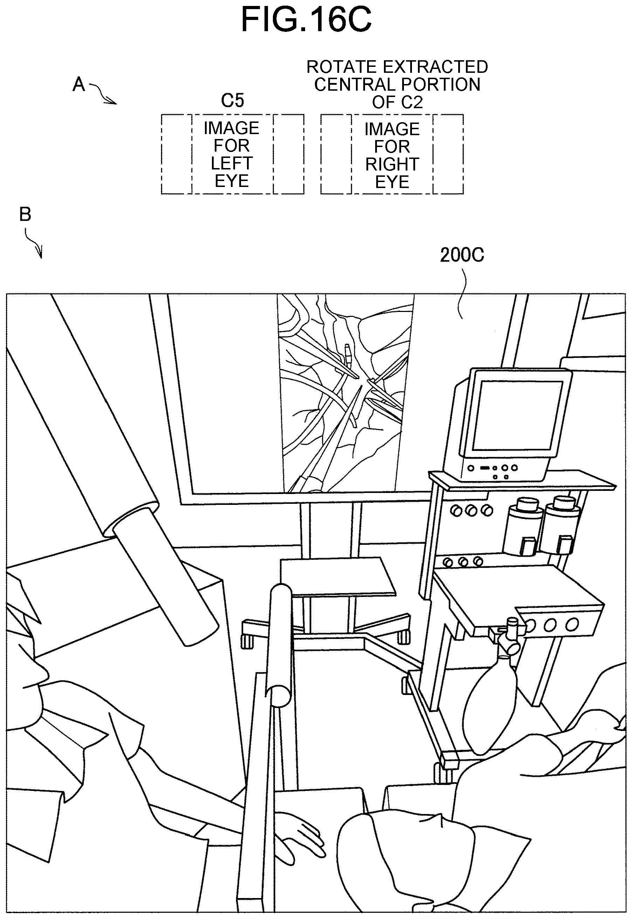

FIG. 15 is a view illustrating an example of a surgical operation field where the medical observation system 1000 according to the present embodiment is used, which is a view illustrating the surgical operation field illustrated in FIG. 1 from another angle. FIG. 16A is a view illustrating an example of a first imaging device and a second imaging device selected by the imaging control process according to the present embodiment, and a medical image displayed on the display device 200A. FIG. 16B is a view illustrating an example of a first imaging device and a second imaging device selected by the imaging control process according to the present embodiment, and a medical image displayed on the display device 200B. FIG. 16C is a view illustrating an example of a first imaging device and a second imaging device selected by the imaging control process according to the present embodiment, and a medical image displayed on the display device 200C.

As illustrated in FIG. 16A, the display device 200A displays a medical image for the left eye captured by the imaging device C1 and a medical image for the right eye captured by the imaging device C3, for example.

As illustrated in FIG. 16B, the display device 200B displays a medical image for the left eye captured by the imaging device C1 and a medical image for the right eye captured by the imaging device C2. That is, the display device 200B displays the medical image for the right eye and the medical image for the left eye having smaller inward angles than in the case of the display device 200A.

As illustrated in FIG. 16C, the display device 200C displays the medical image for the right eye and the medical image for the left eye illustrated with reference to FIG. 14, for example.

The medical observation apparatus 100 selects the first imaging device and the second imaging device by the second imaging control process in the above-described (1) for each of the display devices 200 included in the medical observation system 1000, and then controls to display a medical image on the display screen by the display control process according to the second example as illustrated with reference to FIG. 14, for example.

Accordingly, the medical observation apparatus 100 can control to display an optimal medical image with a more natural three-dimensional effect for each of the display devices 200 included in the medical observation system 1000, as illustrated in FIGS. 16A to 16C, for example.

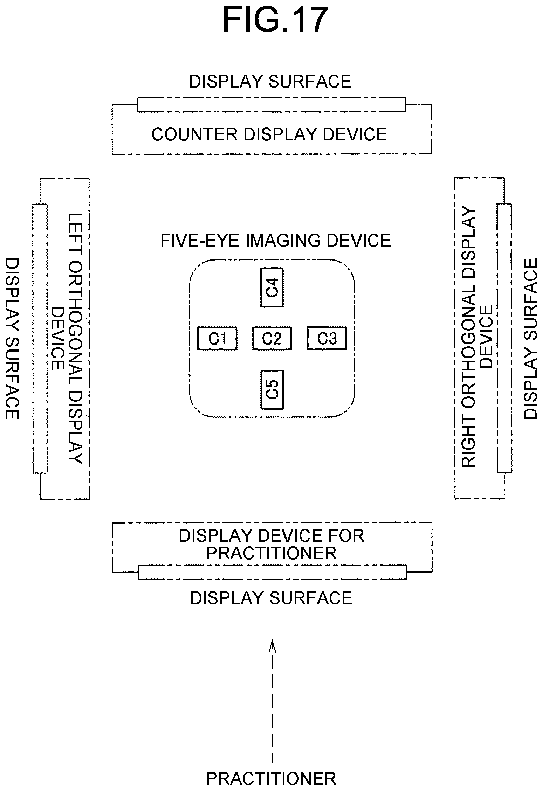

FIG. 17 is a view conceptually illustrating an example of arrangement of the display device 200 in the medical observation system 1000. In FIG. 17, the direction in which the practitioner performs medical practice on the patient is set as a reference direction. In FIG. 17, the display device 200 closer to the practitioner among the display devices 200 arranged in front of the reference direction is referred to as a "practitioner display device", while the display device 200 farther from the practitioner among the display devices 200 arranged in front of the reference direction is referred to as a "counter display device". In FIG. 17, the display device 200 arranged in the left orthogonal direction with respect to the reference direction is referred to as a "left orthogonal display device", while the display device 200 arranged in the right orthogonal direction with respect to the reference direction is referred to as a "right orthogonal display device".

Hereinafter, an example of the process according to the control method of the present embodiment will be described with an example of "displaying the medical image for the right eye and the medical image for the left eye captured by imaging devices C1, C2, C3, C4, and C5 similar to the devices in FIG. 14 on the display screen of the display device 200 arranged as illustrated in FIG. 17". That is, the following example of the processing according to the control method of the present embodiment is an example of the second imaging control process and the display control process according to the second example.

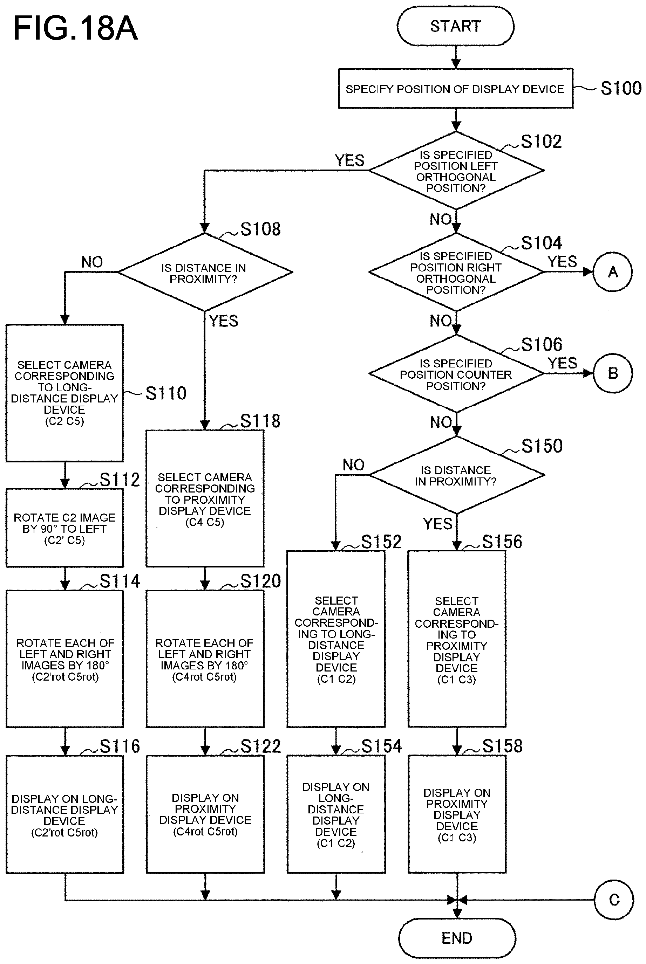

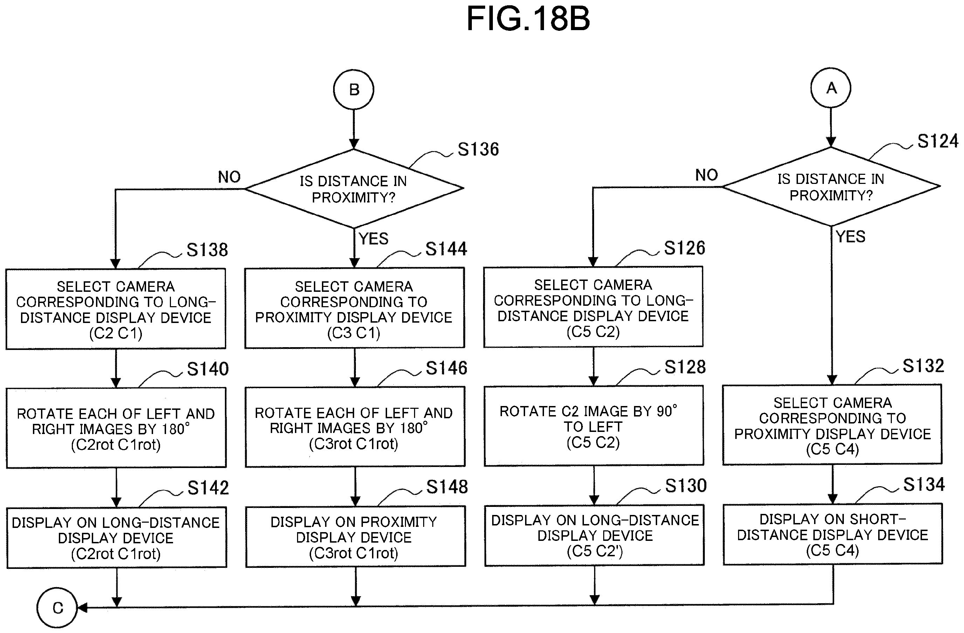

FIGS. 18A and 18B are flowcharts illustrating an example of a process according to the control method of the present embodiment. The process in FIGS. 18A and 18B is performed for each of the display devices 200 that display the medical image for the right eye and the medical image for the left eye. The process illustrated in FIGS. 18A and 18B is performed by the imaging control unit 160 and the display control unit 164 illustrated in FIG. 4, for example. In FIGS. 18A and 18B, one of the imaging devices C1, C2, C3, C4, and C5 is indicated as a "camera". In FIGS. 18A and 18B, "medical images captured by the respective imaging devices C1, C2, C3, C4, and C5" are respectively referred to as "C1," "C2," "C3," "C4," and "C5". In FIGS. 18A and 18B, "an image obtained by rotating the medical image captured by the imaging device C2 by 90[°] to the left" is indicated as "C2'". In FIG. 18A and FIG. 18B, "medical images obtained by rotating the medical images captured by the respective imaging devices C1, C2, C3, C4, and C5 by 180[°]" are respectively referred to as "C1rot", "C2rot", "C3rot", "C4rot", and "C5rot". In FIGS. 18A and 18B, "an image obtained by rotating the medical image captured by the imaging device C2 by 90[°] to the left and is then further rotated by 180[°]" is referred to as "C2'rot".

The medical observation apparatus 100 specifies the position of the display device 200 as a processing target (S100).

The medical observation apparatus 100 refers to "a table (or a database, similar applies hereinafter) associating data specifying the display device 200 (for example, ID of the display device 200) stored in a recording medium that functions as a storage unit (not illustrated) with data indicating arrangement with respect to the reference direction", for example, and thereby specifies the position of the display device 200 as a processing target.

Furthermore, the medical observation apparatus 100 may specify the position of the display device 200 by using any algorithm capable of specifying the position of the display device 200 as a processing target on the basis of data specifying the display device 200 as a processing target, for example. As an example, in a case where the display device 200 as a processing target includes a direction sensor capable of specifying a direction, such as a position sensor and a gyro sensor, the medical observation apparatus 100 acquires data indicating detection results of various sensors (an example of data specifying the display device 200 as a processing target) from the display device 200 as a processing target. Subsequently, the medical observation apparatus 100 specifies the position with respect to the reference direction on the basis of the acquired data indicating the detection results of the various sensors. The medical observation apparatus 100 may specify a viewing distance in the display device 200 as a processing target on the basis of the acquired data indicating the detection results of the various sensors.

The medical observation apparatus 100 determines whether the position specified in step S100 is the left orthogonal position (S102), the specified position is the right orthogonal position (S104), or the specified position is the counter position (S106).

In a case where the specified position is the left orthogonal position, the display device 200 as a processing target corresponds to the "left orthogonal display device" illustrated in FIG. 17. In a case where the specified position is the right orthogonal position, the display device 200 as a processing target corresponds to the "right orthogonal display device" illustrated in FIG. 17. In a case where the specified position is the counter position, the display device 200 as a processing target corresponds to the "counter display device" illustrated in FIG. 17. In a case where the position specified in step S100 does not correspond to the left orthogonal position, the right orthogonal position, or the counter position, the display device 200 as a processing target corresponds to the "practitioner display device" illustrated in FIG. 17.

The order for determining the position is not limited to the example illustrated in FIG. 18A.

### (i) Process in a case where it is determined that the specified position is the left orthogonal position

In a case where it is determined that the position specified in the process of step S102 is the left orthogonal position, the medical observation apparatus 100 determines whether the viewing distance of the display device 200 as a processing target is in proximity (S108). Hereinafter, the display device 200 as a processing target that is determined to have a viewing distance in proximity will be referred to as a "proximity display device" in some cases. In the following, the display device 200 as a processing target that is not determined to have a viewing distance in proximity will be referred to as a "long-distance display device" in some cases.

The medical observation apparatus 100 refers to, for example, a "table (database) associating data specifying the display device 200 (for example, the ID of the display device 200) stored in a recording medium that functions as a storage unit (not illustrated) with data indicating the set viewing distance (for example, a flag indicating whether the device is in proximity or a data indicating the viewing distance as a numerical value)" and thereby specifies the viewing distance set for the display device 200 as a processing target. Subsequently, the medical observation apparatus 100 determines whether the display device 200 as a processing target is in proximity on the basis of the specified viewing distance.

For example, in a case where the viewing distance set for the display device 200 as a processing target is represented by a flag indicating whether the medical device is in proximity, the medical observation apparatus 100 determines whether the display device 200 as a processing target is in proximity following the value indicated by the flag. Furthermore, for example, in a case where the viewing distance set for the processing target display device 200 as a processing target is represented by a numerical value, the medical observation apparatus 100 determines whether the display device 200 as a processing target is in proximity by a threshold process using a set threshold.

As described above, the medical observation apparatus 100 may specify the viewing distance in the display device 200 as a processing target on the basis of the data indicating the detection results of the various sensors acquired from the display device 200 as a processing target and may thereby determine whether the display device 200 as a processing target is in proximity.

In step S108, in a case where the viewing distance of the display device 200 as a processing target is not determined to be in proximity, the medical observation apparatus 100 selects the imaging devices C2 and C5 corresponding to the long-distance display device, and captures medical images using the selected imaging devices C2 and C5 (S110). The medical observation apparatus 100 sets the medical image captured by the imaging device C5 as a medical image for the left eye and sets the medical image captured by the imaging device C2 as a medical image for the right eye. At this time, the medical observation apparatus 100 may perform a "clipping process of clipping a same range for each of the medical image captured by the imaging device C2 and the medical image captured by the imaging device C5".

The medical observation apparatus 100 rotates the medical image captured by the imaging device C2 by 90[°] to the left (S112). Subsequently, the medical observation apparatus 100 rotates each of the medical image for the left eye and the medical image for the right eye by 180[°] (S114) and displays each of the medical images rotated in step S114 on the display device 200 as a processing target (S116).

Moreover, in a case where the viewing distance of the display device 200 as a processing target is determined to be in proximity in step S108, the medical observation apparatus 100 selects the imaging devices C4 and C5 corresponding to the short-distance display device and captures medical images using the selected imaging devices C4 and C5 (S118). The medical observation apparatus 100 sets the medical image captured by the imaging device C5 as a medical image for the left eye and sets the medical image captured by the imaging device C4 as a medical image for the right eye. At this time, the medical observation apparatus 100 may perform a "clipping process of clipping a same range for each of the medical image captured by the imaging device C4 and the medical image captured by the imaging device C5". Subsequently, the medical observation apparatus 100 rotates each of the medical image for the left eye and the medical image for the right eye by 180[°] (S120) and displays each of the medical images rotated in step S120 on the display device 200 as a processing target (S122).

### (ii) Process in a case where it is determined that the specified position is the right orthogonal position

In a case where it is determined that the position specified in the process of step S104 is the right orthogonal position, the medical observation apparatus 100 determines whether the viewing distance of the display device 200 as a processing target is in proximity (S124), similarly to step S108.

In step S124, in a case where the viewing distance of the display device 200 as a processing target is not determined to be in proximity, the medical observation apparatus 100 selects the imaging devices C2 and C5 corresponding to the long-distance display device, and captures medical images using the selected imaging devices C2 and C5 (S126). The medical observation apparatus 100 sets the medical image captured by the imaging device C5 as a medical image for the left eye and sets the medical image captured by the imaging device C2 as a medical image for the right eye. At this time, the medical observation apparatus 100 may perform a "clipping process of clipping a same range for each of the medical image captured by the imaging device C2 and the medical image captured by the imaging device C5".

The medical observation apparatus 100 rotates the medical image captured by the imaging device C2 by 90[°] to the left (S128). Subsequently, the medical observation apparatus 100 controls to display the medical image captured by the imaging device C5 as a medical image for the left eye and sets the medical image rotated in step S128 as a medical image for the right eye on the display device 200 as a processing target (S130).

Moreover, in step S124, in a case where the viewing distance of the display device 200 as a processing target is determined to be in proximity, the medical observation apparatus 100 the medical observation apparatus 100 selects the imaging devices C4 and C5 corresponding to the short-distance display device and captures medical images using the selected imaging devices C4 and C5 (S132). The medical observation apparatus 100 sets the medical image captured by the imaging device C5 as a medical image for the left eye and sets the medical image captured by the imaging device C4 as a medical image for the right eye. At this time, the medical observation apparatus 100 may perform a "clipping process of clipping a same range for each of the medical image captured by the imaging device C4 and the medical image captured by the imaging device C5". Subsequently, the medical observation apparatus 100 controls to display each of the medical image for the left eye and the medical image for the right eye on the display device 200 as a processing target (S134).

### (iii) Processing in a case where it is determined that the specified position is the counter position

In a case where it is determined that the position specified in the process of step S104 is the counter position, the medical observation apparatus 100 determines whether the viewing distance of the display device 200 as a processing target is in proximity (S136), similarly to step S108.

In step S136, in a case where the viewing distance of the display device 200 as a processing target is not determined to be in proximity, the medical observation apparatus 100 selects the imaging devices C1 and C2 corresponding to the long-distance display device and captures medical images using the selected imaging devices C1 and C2 (S138). The medical observation apparatus 100 sets the medical image captured by the imaging device C1 as a medical image for the left eye and sets the medical image captured by the imaging device C2 as a medical image for the right eye. At this time, the medical observation apparatus 100 may perform a "clipping process of clipping a same range for each of the medical image captured by the imaging device C1 and the medical image captured by the imaging device C2". Subsequently, the medical observation apparatus 100 rotates each of the medical image for the left eye and the medical image for the right eye by 180[°] (S140) and displays each of the medical images rotated in step S140 on the display device 200 as a processing target (S142).

Moreover, in step S136, in a case where the viewing distance of the display device 200 as a processing target is determined to be in proximity, the medical observation apparatus 100 selects the imaging devices C1 and C3 corresponding to the short-distance display device and captures medical images using the selected imaging devices C1 and C3 (S144). The medical observation apparatus 100 sets the medical image captured by the imaging device C1 as a medical image for the left eye and sets the medical image captured by the imaging device C3 as a medical image for the right eye. At this time, the medical observation apparatus 100 may perform a "clipping process of clipping a same range for each of the medical image captured by the imaging device C1 and the medical image captured by the imaging device C3". Subsequently, the medical observation apparatus 100 rotates each of the medical image for the left eye and the medical image for the right eye by 180[°] (S146) and displays each of the medical images rotated in step S146 on the display device 200 as a processing target (S148).

### (iv) Process in a case where the specified position does not correspond to the left orthogonal position, the right orthogonal position, or the counter position

In a case where the specified position does not correspond to the left orthogonal position, the right orthogonal position, or the counter position in the process of steps S102, S104, and S106, the medical observation apparatus 100 determines whether the viewing distance of the display device 200 as a processing target is in proximity (S150), similarly to step S108.

In step S150, in a case where the viewing distance of the display device 200 as a processing target is not determined to be in proximity, the medical observation apparatus 100 selects the imaging devices C1 and C2 corresponding to the long-distance display device and captures medical images using the selected imaging devices C1 and C2 (S152). The medical observation apparatus 100 sets the medical image captured by the imaging device C1 as a medical image for the left eye and sets the medical image captured by the imaging device C2 as a medical image for the right eye. At this time, the medical observation apparatus 100 may perform a "clipping process of clipping a same range for each of the medical image captured by the imaging device C1 and the medical image captured by the imaging device C2". Subsequently, the medical observation apparatus 100 controls to display each of the medical image for the left eye and the medical image for the right eye on the display device 200 as a processing target (S154).

Moreover, in step S150, in a case where the viewing distance of the display device 200 as a processing target is determined to be in proximity, the medical observation apparatus 100 selects the imaging devices C1 and C3 corresponding to the short-distance display device, and captures medical images using the selected imaging devices C1 and C3 (S156). The medical observation apparatus 100 sets the medical image captured by the imaging device C1 as a medical image for the left eye and sets the medical image captured by the imaging device C3 as a medical image for the right eye. At this time, the medical observation apparatus 100 may perform a "clipping process of clipping a same range for each of the medical image captured by the imaging device C1 and the medical image captured by the imaging device C3". Subsequently, the medical observation apparatus 100 controls to display each of the medical image for the left eye and the medical image for the right eye on the display device 200 as a processing target (S158).

The medical observation apparatus 100 performs the process illustrated in FIGS. 18A and 18B, for example, as the process according to the control method of the present embodiment and thereby controls to display an optimal medical image with a more natural three-dimensional effect on each of the display devices 200. Needless to say, the example of the processing according to the control method of the present embodiment is not limited to the examples illustrated in FIGS. 18A and 18B.

### [3] An example of an effect achieved by using the control method of the present embodiment

The following effects can be obtained by using the control method according to the present embodiment, for example. Needless to say, the effects achieved by using the control method according to the present embodiment are not limited to the examples described below.
- Realizing natural three-dimensional effect with less fatigue by achieving a match between the inward angle at the time of imaging and the convergence angle at the time of viewing,
- For example, as illustrated in FIGS. 12 and 13, the imaging device unit including four or more perpendicularly arranged imaging devices captures a medical image for the right eye and a medical image for the left eye, thereby making it possible to provide a practitioner and an assistant at a position orthogonal to each other with good stereoscopic images.
- For example, as illustrated in FIGS. 14 to 18B, it is possible to provide an optimal stereoscopic image corresponding to the arrangement of the display device in the medical observation system.
- For example, as illustrated in FIG. 9, coaxial imaging between an overhead view and an enlarged view can be realized by an imaging device unit in which three or more imaging devices are arranged on a same plane.
- Although fine adjustment of the convergence angle of about ± 1[°] is possible in parallax adjustment, it is not possible to cope with a case where the inward angle differs greatly to the extent that the adjustment exceeds the range of the fine adjustment. With respect to this issue, in a case where the control method according to the present embodiment is used, the inward angle can be adjusted to the convergence angle even in a case where the convergence angle is greatly different, making it possible to reproduce the three-dimensional effect with less fatigue.

### (Program according to the present embodiment)

Execution of a program (for example, a program capable of executing processes according to the control method of the present embodiment, such as an "imaging control process", an "imaging control process, or a display control process") for causing a computer system to function as a medical observation apparatus according to the present embodiment (or a control apparatus according to the present embodiment) by a processor or the like in a computer system, thereby enabling acquisition of a medical image with a more natural three-dimensional effect. Here, the computer system according to the present embodiment includes a single computer or a plurality of computers. A series of processes according to the control method of the present embodiment are performed by the computer system according to the present embodiment.

Furthermore, execution of a program for causing a computer system to function as the medical observation apparatus according to the present embodiment (or the control apparatus according to the present embodiment) by a processor or the like in the computer system, making it possible to obtain the effect achieved by the display realized by the processing according to the control method of the present embodiment described above.

The preferred embodiments of the present disclosure have been described above in detail with reference to the accompanying drawings, although the technical scope of the present disclosure is not limited to such examples. It is apparent that a person skilled in the art of the technical field of the present disclosure can make various changes or modifications within the scope of the technical concept described in the claims, and it is naturally understood that these also belong to the technical scope of the present disclosure.

For example, while in the above description, a program (computer program) for causing a computer system to function as the medical observation apparatus according to the present embodiment is provided, the present embodiment can further provide a recording medium storing the above-described program.

The configuration described above illustrates an example of the present embodiment, and naturally belongs to the technical scope of the present disclosure.

Furthermore, the effects described in the present specification are merely illustrative or exemplary and are not limited. That is, the technology according to the present disclosure can exhibit other effects that are apparent to those skilled in the art from the description of the present specification in addition to or instead of the above effects.

Note that the following configurations also belong to the technical scope of the present disclosure.
(1) A medical control apparatus including an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye.
(2) The medical control apparatus according to (1), wherein the imaging control unit adjusts the inward angle on the basis of a convergence angle formed by a right eye and a left eye of a viewer of a display screen that displays the medical image for the right eye and the medical image for the left eye.
(3) The medical control apparatus according to (1) or (2), wherein the imaging control unit changes a baseline length between the first imaging device and the second imaging device and thereby adjusts the inward angle to achieve a match with a convergence angle formed by a right eye and a left eye of a viewer of a display screen that displays the medical image for the right eye and the medical image for the left eye corresponding to the display device.
(4) The medical control apparatus according to any one of (1) to (3), wherein the imaging control unit controls an adjustment mechanism that adjusts the inward angle and thereby adjusts the inward angle.
(5) The medical control apparatus according to (4), wherein
   the adjustment mechanism is a first movement mechanism capable of moving one or both of the first imaging device and the second imaging device, and
   the imaging control unit controls movement of one or both of the first imaging device and the second imaging device by the first movement mechanism.
(6) The medical control apparatus according to (4), wherein
   the adjustment mechanism is a second movement mechanism capable of changing one or both of the imaging direction of the first imaging device and the imaging direction of the second imaging device by moving an optical system, and
   the imaging control unit controls movement of the optical system in the second movement mechanism.
(7) The medical control apparatus according to any one of (4) to (6), wherein the imaging control unit adjusts the inward angle so as to correspond to the display device.
(8) The medical control apparatus according to any one of (4) to (7), further including a display control unit that controls to display the medical image for the right eye and the medical image for the left eye captured after adjustment of the inward angle, on a display screen of the display device.
(9) The medical control apparatus according to any one of (1) to (3), wherein the imaging control unit selects the first imaging device and the second imaging device from three or more imaging devices having different imaging directions and thereby adjusts the inward angle.
(10) The medical control apparatus according to (9), wherein the imaging control unit selects the first imaging device and the second imaging device from the three or more imaging devices arranged on a same plane.
(11) The medical control apparatus according to (10), wherein the imaging control unit selects the first imaging device and the second imaging device so as to correspond to the display device.
(12) The medical control apparatus according to (9), wherein
   three or more imaging devices are arranged on a first plane, and three or more imaging devices are arranged on a second plane orthogonal to the first plane, and
   the imaging control unit selects the first imaging device and the second imaging device from the three or more imaging devices arranged on the first plane or from the three or more imaging devices arranged on the second plane so as to correspond to the display device.
(13) The medical control apparatus according to any one of (9) to (12), further including a display control unit that controls to display the medical image for the right eye captured by the selected first imaging device and the medical image for the left eye captured by the selected second imaging device, on a display screen of the display device.
(14) The medical control apparatus according to (13), wherein
   the display control unit
   selectively performs a rotation process of rotating the medical image for the right eye and the medical image for the left eye so as to correspond to the display device, and
   controls to display the medical image for the right eye and the medical image for the left eye on which the rotation process has been selectively performed, on a display screen of the display device.
(15) The medical control apparatus according to (13) or (14), wherein
   the display control unit
   selectively performs a clipping process of clipping a portion of the medical image for the right eye and a portion of the medical image for the left eye so as to correspond to the display device, and
   controls to display the medical image for the right eye and the medical image for the left eye on which the clipping process has been selectively performed, on a display screen of the display device.
(16) A medical observation system including:
   a medical control apparatus including an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye; and
   a display device that displays the medical image for the right eye and the medical image for the left eye on a display screen.

### Reference Signs List

- 100: MEDICAL OBSERVATION APPARATUS
- 102: BASE
- 104: ARM
- 106, C1, C2, C3, C4, C5: IMAGING DEVICE
- 110a, 110b, 110c, 110d, 110e,: 110f JOINT
- 112a, 112b, 112c, 112d, 112e, 112f: LINK
- 120: IMAGING MEMBER
- 122: TUBULAR MEMBER
- 124: ZOOM SWITCH
- 126: FOCUS SWITCH
- 128: OPERATION MODE CHANGE SWITCH
- 152: ARM UNIT
- 154: IMAGING UNIT
- 156: COMMUNICATION UNIT
- 158: CONTROL UNIT
- 160: IMAGING CONTROL UNIT
- 162: ARM CONTROL UNIT
- 164: DISPLAY CONTROL UNIT
- 200, 200A, 200B, 200C, 200D: DISPLAY DEVICE
- 1000: MEDICAL OBSERVATION SYSTEM

## Claims

1. A medical control apparatus comprising an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye.

2. The medical control apparatus according to claim 1, wherein the imaging control unit adjusts the inward angle on the basis of a convergence angle formed by a right eye and a left eye of a viewer of a display screen that displays the medical image for the right eye and the medical image for the left eye.

3. The medical control apparatus according to claim 1, wherein the imaging control unit changes a baseline length between the first imaging device and the second imaging device and thereby adjusts the inward angle to achieve a match with a convergence angle formed by a right eye and a left eye of a viewer of a display screen that displays the medical image for the right eye and the medical image for the left eye corresponding to the display device.

4. The medical control apparatus according to claim 1, wherein the imaging control unit controls an adjustment mechanism that adjusts the inward angle and thereby adjusts the inward angle.

5. The medical control apparatus according to claim 4, wherein
the adjustment mechanism is a first movement mechanism capable of moving one or both of the first imaging device and the second imaging device, and
the imaging control unit controls movement of one or both of the first imaging device and the second imaging device by the first movement mechanism.

6. The medical control apparatus according to claim 4, wherein
the adjustment mechanism is a second movement mechanism capable of changing one or both of the imaging direction of the first imaging device and the imaging direction of the second imaging device by moving an optical system, and
the imaging control unit controls movement of the optical system in the second movement mechanism.

7. The medical control apparatus according to claim 4, wherein the imaging control unit adjusts the inward angle so as to correspond to the display device.

8. The medical control apparatus according to claim 4, further comprising a display control unit that controls to display the medical image for the right eye and the medical image for the left eye captured after adjustment of the inward angle, on a display screen of the display device.

9. The medical control apparatus according to claim 1, wherein the imaging control unit selects the first imaging device and the second imaging device from three or more imaging devices having different imaging directions and thereby adjusts the inward angle.

10. The medical control apparatus according to claim 9, wherein the imaging control unit selects the first imaging device and the second imaging device from the three or more imaging devices arranged on a same plane.

11. The medical control apparatus according to claim 10, wherein the imaging control unit selects the first imaging device and the second imaging device so as to correspond to the display device.

12. The medical control apparatus according to claim 9, wherein
three or more imaging devices are arranged on a first plane, and three or more imaging devices are arranged on a second plane orthogonal to the first plane, and
the imaging control unit selects the first imaging device and the second imaging device from the three or more imaging devices arranged on the first plane or from the three or more imaging devices arranged on the second plane so as to correspond to the display device.

13. The medical control apparatus according to claim 9, further comprising a display control unit that controls to display the medical image for the right eye captured by the selected first imaging device and the medical image for the left eye captured by the selected second imaging device, on a display screen of the display device.

14. The medical control apparatus according to claim 13, wherein
the display control unit
selectively performs a rotation process of rotating the medical image for the right eye and the medical image for the left eye so as to correspond to the display device, and
controls to display the medical image for the right eye and the medical image for the left eye on which the rotation process has been selectively performed, on a display screen of the display device.

15. The medical control apparatus according to claim 13, wherein
the display control unit
selectively performs a clipping process of clipping a portion of the medical image for the right eye and a portion of the medical image for the left eye so as to correspond to the display device, and
controls to display the medical image for the right eye and the medical image for the left eye on which the clipping process has been selectively performed, on a display screen of the display device.

16. A medical observation system comprising:
a medical control apparatus including an imaging control unit that adjusts an inward angle which is an angle formed by an imaging direction in a first imaging device that captures a medical image for a right eye and an imaging direction in a second imaging device that captures a medical image for a left eye to the inward angle corresponding to a display device that displays the medical image for the right eye and the medical image for the left eye; and
a display device that displays the medical image for the right eye and the medical image for the left eye on a display screen.
